# EUROPEAN PATENT APPLICATION

(11) **EP 4 219 684 A1**
(43) Date of publication of application: **02.08.2023**
(21) Application number: 21871593.6
(22) Date of filing: 24.09.2021
(51) Int. Cl.: C12N 5/0735

(54) **ACTIVATED PLURIPOTENT STEM CELL, AND PREPARATION METHOD THEREFOR AND USE THEREOF**

(30) Priority: 24.09.2020 CN 202011016234
(71) Applicant: Institute Of Zoology, Chinese Academy Of Sciences, Beijing 100101 (CN); Beijing Institute for Stem Cell and Regenerative Medicine, Beijing 100101 (CN)
(72) Inventor: LI, Lei, Beijing 100101 (CN); WANG, Xiaoxiao, Beijing 100101 (CN); WANG, Qizhi, Beijing 100101 (CN)
(74) Representative: Lavoix
(86) International application number: PCT/CN2021/120283
(87) International publication number: WO 2022/063224

(57) **Abstract**

A pluripotent stem cell, a method for producing the pluripotent stem cell, and the use of the pluripotent stem cell for stem cell differentiation, cell transplantation, tissue repair, and/or tissue regeneration.

## Description

### TECHNICAL FIELD

The present application relates to the field of pluripotent stem cells. Specifically, the present application relates to a formative pluripotent stem cell, a method for producing the pluripotent stem cell, and the use of the pluripotent stem cell for stem cell differentiation, cell transplantation, tissue repair and/or tissue regeneration.

### BACKGROUND ART

During the pre-implantation to gastrulation process of mammalian embryonic development, epiblast cells have the potential to differentiate into all cells in the body. Such cells with pluripotency can be cultured in vitro to obtain embryonic stem cells and epiblast cell lines. In addition, somatic cells can be induced to establish induced pluripotent stem cells. These cells can produce cells with various functions through a variety of complex induction methods, including: germ cells and somatic cells; provide potential medical materials for cell, tissue and organ transplantation and regeneration, and provide a nice model for drug screening of related diseases. However, in the process of differentiation of embryonic stem cells and induced pluripotent stem cells into functional cells, the efficiency of cell differentiation, especially the poor efficiency of directed differentiation, is a key problem in the field; in addition, the heterogeneity of cell differentiation is also another of the most important issues facing the field.

At present, it is considered that embryonic stem cells (ESCs) and induced pluripotent stem cells (iPSCs) are in a naive state and have not yet reached the state of ready for differentiation, thus it takes a long time to differentiate into various types of cells, and differentiation effects in different laboratories or under different conditions are quite different. In particular, in the process of differentiation from embryonic stem cells to germ cells, epiblast-like cells (EpiLCs)-an unstable intermediate state must be gone through before they can be more effectively differentiated into germ cells. The epiblast cell (EpiSC) line is in a primed state, losing some characteristics of early epiblast cells in vivo, and cannot effectively differentiate into germ cells in vitro; in addition, epiblast cell lines established in different laboratories are also very different and have not been widely used.

During embryonic development, although epiblast cells exist only transiently before and after gastrulation in vivo, they can differentiate into cells of various lineages, including germ cells. A lot of recent experimental evidence shows that there may be a third state-the formative state-between the naive and primed pluripotent state. This hypothesis holds that during epiblast development, naive cells need to go through "capacitation" to reach an activated state with the ability to differentiate into various cells, and speculate on some molecular characteristics of cells in this state. During the development of mammalian epiblast cells, if there is an activated state, especially if the state can be stably maintained in vitro, they are called formative pluripotent stem cells (fPSCs). Compared with other pluripotent stem cell lines, fPSCs may have obvious advantages in differentiation and application. However, there are no reports of any in vitro stable formative pluripotent stem cell lines.

### SUMMARY OF THE PRESENT APPLICATION

After a large number of experiments and repeated explorations, the inventors of the present application obtained a method of obtaining a pluripotent stem cell line (a formative pluripotent stem cell (fPSC) line) about to undergo gastrulation, comparing to the embryonic stem cells and other pluripotent stem cells that have been reported so far, the formative pluripotent stem cell line obtained by this method has significantly improved differentiation efficiency and differentiation potential, and has great application value.

### Formative pluripotent stem cells (fPSCs)

The first aspect of the present application provides an isolated pluripotent stem cell-fPSC, which can self-renew and can maintain cell properties without significant changes in multiple passages.

The fPSCs of the present application have functions and/or characteristics similar to or imitating mammalian embryonic epiblast cells from post-implantation to about to undergo gastrulation, in particular, the pluripotent stem cells of the present application have pluripotency of mammalian embryonic epiblast cells from post-implantation to about to undergo gastrulation.

In certain embodiments, the epiblast cells are mammalian embryonic epiblast cells from post-implantation to about to undergo gastrulation. In certain embodiments, the mammalian embryonic epiblast cells from post-implantation to about to undergo gastrulation refer to the epiblast cells corresponding to the embryonic developmental stage of mouse embryo E5.5-E6.5. In certain embodiments, the pluripotency of the mammalian embryonic epiblast cells from post-implantation to about to undergo gastrulation includes the ability to differentiate into endoderm, mesoderm and ectoderm cells, and primordial germ cells (PGCs).

The fPSCs of the present application are in a formative state between naive and primed pluripotent states. For a detailed description of the formative state, see, for example, Smith A. Development. 2017 Feb 1; 144(3): 365-373. The so-called formative state means that during the development of the epiblast, naive stem cells need to undergo "capacitation" to reach a state with the ability to differentiate into various cells. During the development of naive epiblast cells, the process of the formative state cells need to experience, including: the establishment of specific epigenetic modifications at the DNA and chromatin levels, the formation of unique gene regulatory networks, the regulation of important signaling pathways, and the changes of important metabolism pathways (e.g., glycolysis), etc. Furthermore, partial epithelialization and increased interaction with the extracellular matrix are expected to modulate signaling efficiency. In terms of overall gene expression levels, fPSCs have a transcriptional state intermediate between naive and primed pluripotent stem cells. Compared with naive pluripotent stem cells, fPSCs express low levels of naive markers; compared with primed pluripotent stem cells, fPSCs express low levels of multiple cell lineage markers; in addition, fPSCs have own unique markers. fPSCs require a comprehensive remodeling of their chromatin landscape. fPSCs have lost their naive properties and acquired the ability of gamete and somatic cell fate selection. Thus, they respond more rapidly and consistently to inductive signals than naive cells. Mouse embryonic E5.5-E6.5 epiblast cells have the characteristics of formative cells.

The formative state described herein includes those distinctly different from naive and primed pluripotent states, including at least one selected from the following:
(1) it has the differentiation ability similar to that of mammalian epiblast cells about to undergo gastrulation, that is, has the ability to differentiate to the endoderm, mesoderm and ectoderm cells, and primordial germ cells (PGCs);
(2) compared with naive ESCs, it is more rapid and efficient to respond to signals that induce cell lineage differentiation. For example, compared with naive ESCs, the response speed and differentiation efficiency of formative ESCs to the signal inducing cell lineage differentiation are improved by at least 10%, at least 20%, at least 30%, at least 40%, at least 50%, at least 60%, at least 70%, at least 80%, at least 90%, at least 100%, at least 150%, at least 200% or more.
(3) it has a rosette-like structure and/or a polar cavity that can be marked by Ezrin immunostaining. In some cases, the formative state may further include the morphological characteristics described above.
(4) compared with the native and primed states, it highly expresses formative markers, in some cases, the formative state may further include at least one genome or epigenome feature selected from the following: e.g., OTX2, DNMT3B, SOX3/4, FGF5, ZIC2/5, ETV1/4, GRHL2, LIN28B, UTF1, ZSCAN10, FGF8, ERAS, ESRP1, CLDN6/7, etc.;
(5) compared with the naive state, it expresses low levels of naive pluripotent transcription factors, e.g., KLF2/4, TBX3, TFCP2L1, etc.;
(6) compared with the primed state, it expresses low levels of primed important transcription factors, e.g., BRACHYURY/T, GATA6, FOXA2, etc.;
(7) compared with the naive and primed states, it has a high level of chromatin bivalency (in the same region of DNA fragments bound to histones, there are both transcriptional repression H3K27me3 histone modifications and transcriptional activation H3K4me3 histone modification), with a high level of super covalent modified genes, e.g., HOXA CLUSTER, EVX1, GATA4, PAX2, etc.

In certain embodiments, the pluripotent stem cells of the present application are formative pluripotent stem cells, which have at least one of the above characteristics (1) or (2) (e.g., both of them). In certain embodiments, the pluripotent stem cells of the present application further possess the above-mentioned characteristic (3). In certain embodiments, the pluripotent stem cells of the present application further possess at least one of the above characteristics (4)-(7) (e.g., at least 2, at least 3, or all 4).

### I. Functional characterization

In certain embodiments, the pluripotent stem cells of the present application have the following characteristics:
(i) having the pluripotency of mammalian embryonic epiblast cells from post-implantation to about to undergo gastrulation;
(ii) having the differentiation function of the formative state described above, optionally also having the morphological characteristics and/or genome or epigenome characteristics of the formative state described above;
(iii) self-renewable and/or being capable of stabe passage for at least 5times, such as at least 10 times, at least 15 times, at least 20 times, at least 25 times, at least 30 times or more.

In certain embodiments, the pluripotent stem cells of the present application are capable of differentiating into germ cells or germline precursor cells or germline precursor-like cells. In certain embodiments, the pluripotent stem cells of the present application are capable of differentiating into germ cells or germline precursor cells or germline precursor-like cells within 10 days, 9 days, 8 days, 7 days, 6 days, 5 days, 4 days, 3 days, 2 days, or 1 day after application of differentiation conditions. In certain embodiments, the differentiation efficiency is at least 5%, at least 6%, at least 7%, at least 8%, at least 9%, at least 10%, at least 20%, at least 30%, at least 40%, at least 50%, at least 60%, at least 70%, at least 80%, at least 90% or more.

In certain embodiments, the pluripotent stem cells of the present application are capable of differentiating into cells of the mesoderm lineage, examples of which include, but are not limited to: adipogenic, smooth muscle formation, chondrogenic, cardiogenic, dermatogenic, hematopoietic, angiogenic, myogenic, nephrogenic, urogenic, osteogenic, pericardial-derived, or stromal cells. In certain embodiments, the differentiation efficiency is at least 50%, at least 60%, at least 70%, at least 80%, at least 90% or more under conditions that allow differentiation of pluripotent stem cells.

In certain embodiments, the pluripotent stem cells of the present application are capable of differentiating into cells of the ectodermal lineage, examples of which include, but are not limited to, epidermal cells, neuron cells, and glial cells. In certain embodiments, the differentiation efficiency is at least 50%, at least 60%, at least 70%, at least 80%, at least 90% or more under conditions that allow differentiation of pluripotent stem cells.

In certain embodiments, the pluripotent stem cells of the present application are capable of differentiating into cells of the endoderm lineage, examples of which include, but are not limited to, cells producing pancreas, liver, lung, stomach, intestine, and thyroid. In certain embodiments, the differentiation efficiency is at least 50%, at least 60%, at least 70%, at least 80%, at least 90% or more under conditions that allow differentiation of pluripotent stem cells.

In certain embodiments, the pluripotent stem cells of the present application are capable of differentiating into endoderm, ectoderm, or mesoderm cells within five days, four days, three days, two days, or one day of application of differentiation conditions. In certain embodiments, the differentiation efficiency is at least 20%, at least 30%, at least 40%, at least 50%, at least 60%, at least 70%, at least 80%, at least 90% or more.

### II. Marker Characterization

In certain embodiments, compared with the expression level of these genes in an embryonic stem cell, the expression level of one or more (e.g., 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, or all 70) genes selected from the following in the pluripotent stem cell of the present application show at least about 1.5 times, at least about 2 times increase: CLDN6, WNT3, MIXL1, EOMES, TCFL5, ZIC5, LIN28B, GRHL2, MYCN, ABHD14A, RPUSD2, MVK, SPRED2, E2F3, TFAP4, HGH1, PPIF, TFDP1, PRSS8, SLC9A3R1, IFRD2, KDF1, ARRB2, KLHL23, TSPAN5, RAB25, CLDN6, DHCR7, RBM47, KHK, SMCO4, NABP2, RBPMS2, TMEM39B, REPIN1, MVD, SOX3, RARG, TMEM30B, IGSF9, CDH24, LSS, MARVELD3, ADGRB2, EOMES, ZCCHC3, SLC39A8, ZSCAN10, FADS2, NECTIN1, SNAI3, MOXD1, MYC, PDZD4, SLC7A8, ETV4, SEPHS1, ZIC2, JPH4, TMEM125, MPLKIP, LSM2, ZIC5, SLC27A2, MMP25, DERL3, TMEM59L, EPHA1, FOXD3, ETV5.

In certain embodiments, compared with the expression level of these genes in an embryonic stem cell, the expression level of one or more (e.g., 2, 3, 4, 5, 6, 7, 8, 9, 10, 11, 12, 13, 14, 15, 16, 17, or all 18) genes selected from the following in the pluripotent stem cell show at least about 2 times, at least about 3 times, at least about 4 times, at least about 5 times, at least about 6 times, at least about 7 times, at least about 8 times, at least about 9 times, or at least about 10 times increase: ZIC2, ZIC5, ESRP1, UTF1, ETV1, ETV4, GRH12, CLDN7, CLDN6, WNT3, MIXL1, SOX4, LIN28B, MYC, MOXD1, NECTIN1, SLC39A8, SLC7A8. In certain embodiments, compared with the expression level of these genes in an embryonic stem cell, the expression level of one or more (e.g., 2, 3, 4, 5, 6, 7, 8, 9, or all) genes selected from the following in the pluripotent stem cell show at least about 10 times increase: CLDN6, WNT3, MIXL1, SOX4, LIN28B, EPHA1, JPH4, MMP25, TMEM125, TMEM59L.

In certain embodiments, the expression level of EOMES in the pluripotent stem cell is at least about 2 times, at least about 3 times, at least about 4 times or about 5 times that of the gene in an embryonic stem cell. In certain embodiments, the expression level of EOMES in the pluripotent stem cell is about 5 times that of the gene in an embryonic stem cell.

In certain embodiments, the expression level of TCFL5 in the pluripotent stem cell is at least about 2 times, at least about 3 times, at least about 4 times, at least about 5 times, at least about 6 times, at least about 7 times, at least about 8 times, at least about 9 times or about 10 times that of the gene in an embryonic stem cell. In certain embodiments, the expression level of TCFL5 in the pluripotent stem cell is about 5 times to about 10 times that of the gene in an embryonic stem cell.

In certain embodiments, the expression level of ZIC5 in the pluripotent stem cell is at least about 2 times, at least about 3 times, at least about 4 times, at least about 5 times, at least about 6 times, at least about 7 times, at least about 8 times, at least about 9 times or about 10 times that of the gene in an embryonic stem cell. In certain embodiments, the expression level of ZIC5 in the pluripotent stem cell is about 5 times to about 10 times that of the gene in an embryonic stem cell.

In certain embodiments, the expression level of FOXH1 in the pluripotent stem cell is at least about 2 times, at least about 3 times, at least about 4 times or about 5 times that of the gene in an embryonic stem cell. In certain embodiments, the expression level of FOXH1 in the pluripotent stem cell is about 3 times to about 5 times that of the gene in an embryonic stem cell.

In certain embodiments, the expression level of GRHL2 in the pluripotent stem cell is at least about 2 times, at least about 3 times, at least about 4 times or about 5 times that of the gene in an embryonic stem cell. In certain embodiments, the expression level of GRHL2 in the pluripotent stem cell is about 4 times to about 5 times that of the gene in an embryonic stem cell.

Herein, said expression can be monitored by measuring the level of full length mRNA, mRNA fragments, full length protein or protein fragments of the genes. Thus, in certain embodiments, the expression level is mRNA level or protein level. In some embodiments, the expression is assessed by analyzing the expression of mRNA transcripts of the genes, for example, by determining the levels of mRNA of these genes in the cells by RT-PCR. In other embodiments, the expression is assessed by analyzing the expression of protein products of the genes, for example, by determining the levels of the proteins of these genes in the cells by immunological detection.

### III. Epigenetic modification characterization

In certain embodiments, the pluripotent stem cells of the present application comprise at least 200, at least 250, or at least 300 super bivalent genes.

It is known in the art that when used to describe epigenetics, "covalent" (also referred to as bivalent/bivalency) refers to an epigenetic modification with both a transcriptional activation marker H3K4me3 and a transcriptional repression marker H3K27me3, wherein, H3K4me3 refers to the trimethylation modification on the 4th lysine of histone H3, and H3K27me3 refers to the trimethylation modification of the 27th lysine on histone H3. A gene in which the above-mentioned covalent modifications exist in the promoter region is called a bivalent gene. "Super bivalent/super bivalency" refers to a bivalent modification that meets the following conditions in ChIP-seq assays: 1) the enrichment signal of H3K4me3 in the promoter region is stronger than that of the house-keeping gene; 2) the promoter region has strong H3K27me3 modification (RPKM>1 after normalization). Gene with the above-mentioned super bivalent modifications in the promoter region is called a super bivalent gene. Detailed description of super bivalent modification can be found in Xiang Y, et al. Nat Genet. 2020 Jan;52(1):95-105, which is incorporated herein by reference. Herein, when used to describe epigenetics, "bivalent" and "covalent" are two translations of bivalency, both have the same meaning and can be used interchangeably.

In certain embodiments, the pluripotent stem cell has a super bivalency in the promoter region of at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, or 31 genes selected from the following: ABLIM2, ANKRD33B, BARX1, DMRT2, DMRT3, EVX1, FGFR3, FLT1, FOXC1, FOXF2, GDNF, HOXA11, HOXA3, HOXA5, HOXA6, IRX1, IRX2, KISS1R, MSX1, NFIB, NFIC, NKX3-2, NRN1, OTP, PAX5, PHOX2B, PITX3, PTGER4, SLIT2, TBX1, VLDLR.

In certain embodiments, the pluripotent stem cell has a super bivalency in the promoter region of at least 200, at least 250 or at least 300 genes selected from the following: ABLIM2, ADAM22, ADM, ALX4, ANKRD33B, ANKRD34A, AP3M2, ARHGEF26, ARID5A, ATOH1, B4GALNT2, BARHL1, BARHL2, BARX1, BCAN, BCL2L11, BMP7, BSX, C1QL1, C1QL4, CACNA1H, CADPS, CAMK2D, CASZ1, CBLN1, CBX4, CBX8, CCNO, CCR10, CD44, CDH3, CDX2, CEBPD, CHPF, CNNM2, COCH, COL12A1, COL2A1, CRLF1, CSF1, CSMD3, DBNDD1, DBX1, DCLK2, DDHD1, DDN, DHH, DLG5, DLK1, DLL1, DLL4, DLX1, DLX4, DLX5, DLX6, DMRT2, DMRT3, DMRTA2, DPF3, EBF1, EBF2, EBF3, EDA, EFHD1, EFNA3, EGR3, EMX1, EMX2, EN2, EOMES, EPHA7, EPHB1, ESAM, EVX1, FBXL8, FEV, FEZF1, FEZF2, FGF11, FGF8, FGF9, FGFR2, FGFR3, FLI1, FLT1, FOS, FOSB, FOSL2, FOXA1, FOXA2, FOXB1, FOXB2, FOXC1, FOXC2, FOXD3, FOXD4, FOXF2, FOXI3, FOXL1, FOXL2, FZD10, GAD1, GAD2, GATA2, GATA3, GATA4, GATA6, GBX2, GDNF, GFI1, GP1BB, GRIK1, GRIN1, GRK4, GRM1, GRRP1, GSC, GSX1, HAND1, HAND2, HELT, HES5, HES7, HIC1, HLX, HMX2, HMX3, HNF1B, HOXA1, HOXA11, HOXA2, HOXA3, HOXA5, HOXA6, HOXB13, HOXB5, HOXB6, HOXB7, HOXC10, HOXC12, HOXC4, HOXC5, HOXC6, HOXC9, HS3ST3B1, HSD17B1, HSF4, ICAM4, ICAM5, INSM1, IRX1, IRX2, IRX3, IRX5, ISL1, ISL2, ISLR2, JAK3, KAZALD1, KCNK13, KCNQ2, KIRREL2, KIRREL3, KISS1R, LBX1, LEFTY1, LFNG, LHFPL3, LHX1, LHX2, LHX4, LHX5, LHX8, LHX9, LIPG, LIX1L, LMX1A, LMX1B, LRBA, MAB21L1, MAB21L2, MAFB, MAL, MDGA1, MEIS1, MEIS2, MID1IP1, MKX, MNX1, MSX1, MSX2, MTHFSD, NAB2, NCAM1, NEUROG2, NFATC1, NFIA, NFIB, NFIC, NKX1-2, NKX2-2, NKX2-5, NKX3-2, NKX6-1, NKX6-2, NR2E1, NR2F1, NR2F2, NR4A2, NRN1, NRP2, NTN1, NTNG2, NUAK2, NXPH1, NXPH4, OLIG2, ONECUT1, ONECUT2, OSR1, OSR2, OTP, OTX1, PAX1, PAX2, PAX3, PAX5, PAX6, PAX7, PAX8, PAX9, PCDH10, PCDH17, PCDH7, PCDH8, PCDHGC4, PCDHGC5, PCSK5, PDX1, PHF21B, PHOX2B, PITX1, PITX2, PITX3, PLEKHH3, PLXDC1, PORCN, POU3F2, POU4F3, PPM1J, PRDM12, PRDM13, PRDM16, PRDM6, PRDM8, PROK2, PRRX1, PTGER4, PTPRU, PURA, RAPGEF3, RAX, RBM3, RBMS3, RBMX, RCSD1, REPIN1, RHPN1, RTN4RL2, SALL3, SATB2, SCN3B, SCRT1, SEZ6, SFI1, SH3PXD2A, SHH, SHISA9, SHOX2, SIDT1, SIM1, SIM2, SIX1, SIX2, SIX3, SIX6, SKOR1, SLC17A6, SLC25A25, SLC6A5, SLIT2, SOCS3, SOX1, SOX14, SOX17, SOX9, SP8, SP9, SPATA18, SPATA3, SPHK1, SPOCK1, SYNGR1, T, TAL1, TBR1, TBX1, TBX15, TBX2, TBX20, TBX3, TBX4, TCF21, TLX1, TLX3, TMEM102, TNIK, TRADD, TRIM67, TRMT61A, VAV1, VAX1, VAX2, VGF, VLDLR, WNT10B, WNT11, WNT5A, WT1, ZC3HAV1, ZFP46, ZFP503, ZFP536, ZFP703. In some embodiments, the pluripotent stem cells has a super bivalency in the promoter region of at least one or more (e.g., 2, 3, 4, 5, 6, 7, 8, 9, 10, 11 or all 12) genes selected from the following: HAND1, T, FOXA2, NKX2-5, PAX6, PDX1, ISL1, TCF21, LHX5, PAX2, DLX5, NR4A2.

In certain embodiments, compared with the promoter region of the corresponding genes in an embryonic stem cell, the promoter region of one or more (e.g., 2, 3, 4, 5, 6, 7, 8, 9, 10, 11 or all 12) genes selected from the following in the pluripotent stem cell shows a lower DNA methylation level: HANOI, T, FOXA2, NKX2-5, PAX6, PDX1, ISL1, TCF21, LHX5, PAX2, DLX5, NR4A2.

In certain embodiments, compared with the promoter region of the corresponding genes in an embryonic stem cell, the promoter region of one or more (e.g., 2, 3, 4, 5, 6, 7 or all 8) genes selected from the following in the pluripotent stem cell shows an increased H3K4ME3 level: OTX2, ZIC5, UTF1, FGF5, ZFP13, ZSCAN10, ZIC2, ESRP1.

In certain embodiments, compared with the promoter region of the corresponding genes in an embryonic stem cell, the promoter region of one or more (e.g., 2, 3, 4, 5, 6, 7 or all 8) genes selected from the following in the pluripotent stem cell show a decreased H3K27ME3 level: OTX2, ZIC5, UTF1, FGF5, ZFP13, ZSCAN10, ZIC2, ESRP1.

In certain embodiments, compared with the promoter region of the corresponding genes in an embryonic stem cell, the promoter region of one or more (e.g., 2, 3, 4, 5, 6, 7 or all 8) genes selected from the following in the pluripotent stem cell show a decreased DNA methylation level: OTX2, ZIC5, UTF1, FGF5, ZFP13, ZSCAN10, ZIC2, ESRP1.

In certain exemplary embodiments, the above-mentioned epigenetic modification is determined by ChIP-seq method.

In certain embodiments, the pluripotent stem cell of the present application comprises two X chromosomes, one of which is a normally activated X chromosome (Xa), and the other is a normally silent X chromosome (Xi).

In certain embodiments, the embryonic stem cells mentioned in any of the above embodiments (e.g., the embryonic stem cells to which the pluripotent stem cells of the present application are compared) can be established embryonic stem cell lines, for example, certain cell lines that can be obtained from public preservation systems or commercially available cell lines, or those that can be obtained directly from original embryonic tissues.

Methods for obtaining embryonic stem cells from embryonic tissues are well known in the art, for example embryonic stem cells can be isolated from blastocysts of various primate species (US 5,843,780; Thomson et al., Proc. Natl. Acad. Sci. USA 92:7844, 1995), or human embryonic stem (hES) cells are prepared from human blastocyst cells based on the technique described by Thomson et al. (US 6,200,806; Science 282:1145, 1998; Curr. Top. Dev. Biol. 38:133, 1998) and according to Reubinoff et al. (Nature Biotech. 18:399,2000) using primary mouse fibroblast feeder cells, or hES cells lines can also be derived from human feeder cells (US 6,642,048) or derived in the condition of completely feeder cells-free ((US 2002/0081724) or Klimanskaya et al., The Lancet, 365(9471):1636-41 (2005)) conditions.

A number of embryonic stem cell lines that have been identified include but not limited to: H1, H7, H9, H13, and H14 (Thompson et al.); hESBGN-01, hESBGN-02, hESBGN-03 (BresaGen, Inc.); HES-1, HES-2, HES-3, HES-4, HES-5, HES-6 (ES Cell International, Inc.); HSF-1, HSF-6 (University of California, San Francisco); I3, I4, I6 (Israel Institute of Technology); UCSF-1 and UCSF-2 (Genbacev et al., Fertil. Steril. 83(5):1517-29, 2005); HUES 1-17 line (Cowan et al., NEJM 350(13):1353-56, 2004); and ACT-14 line (Klimanskaya et al., Lancet, 365(9471): 1636-41, 2005).

### IV. Morphological features

In certain embodiments, the pluripotent stem cells of the present application are capable of forming an epiblast-like embryoid (EpiBlastoid) with a rosette-like structure.

In certain embodiments, the pluripotent stem cells of the present application are capable of being passaged at least 5 times, e.g., at least 10 times, at least 15 times, at least 20 times, at least 25 times, at least 30 times or more, without significant changes in cell properties. In certain embodiments, the pluripotent stem cells of the present application are capable of being passaged 1-100 times, e.g., 1-90 times, 1-80 times, 1-70 times, 1-60 times, 1-50 times, 1-45 times, 1-40 times, 1-35 times or 1-30 times, without significant changes in cell properties. In certain embodiments, the pluripotent stem cells are capable of maintaining or substantially maintaining the properties described above (including functions, markers, epigenetic modifications and/or morphological characteristics) when passaged.

In certain embodiments, the pluripotent stem cells described in the present application are derived from pluripotent stem cells before and after gastrulation. In certain embodiments, the pluripotent stem cells are derived from or selected from the following cells: embryonic stem cells (ESCs), induced pluripotent stem cells (iPSC), epiblast stem cells (EpiSCs), epiblast cells, somatic cells, pluripotent stem cells induced from somatic cells, somatic stem cells, blastocyst inner cell mass.

In certain embodiments, the epiblast is derived from the epiblast of a mammalian embryo from post-implantation to pre- or post-gastrulation or about to undergo gastrulation, such as mouse embryo E5.5-E6.5 Epiblast, human embryo E12-E14 Epiblast, sheep embryo E13-E15 or bovine embryo E16-E19.

In certain embodiments, the pluripotent stem cells of the present application are derived from mammalian embryos and established pluripotent stem cells, including human and non-human pluripotent stem cells, such as pluripotent stem cells of rodents (e.g., mice and rats), ungulates (e.g., cattle, sheep, goats and pigs), or other mammals (e.g., cats, dogs, horses or rabbits). In certain embodiments, the pluripotent stem cells are mouse or human pluripotent stem cells.

In certain embodiments, the pluripotent stem cells of the present application are cell lines. In certain embodiments, the pluripotent stem cells of the present application are stable cell lines, which are capable of maintaining long-term stability in vitro. In certain embodiments, the pluripotent stem cells of the present application are formative pluripotent stem cell lines.

The second aspect of the present application also provides an isolated cell population comprising the pluripotent stem cells described in the first aspect.

In certain embodiments, at least 50% (e.g., at least 60%, at least 70%, at least 80%, at least 85%, at least 90%, at least 95%, at least 98%, at least 99%, or about 100%) of the cells in the cell population are the pluripotent stem cells described in the first aspect.

In certain embodiments, the pluripotent stem cells are capable of forming the epiblast-like embryoids by culturing (e.g., three-dimensional culture) in a medium selected from basal medium supplemented with: serum substitutes, Activin A, bFGF, and Wnt/β-catenin signal transduction inhibitors.

In certain embodiments, the basal medium is selected from KnockOut DMEM, KnockOut DMEM/F 12, DMEM, DMEM/F 12, neurobasal medium, and any combination thereof.

In certain embodiments, the basal medium is selected from basal medium supplemented with N-2 (e.g., Gibco: Cat. No. 17502048) and B-27 (e.g., Gibco: Cat. No. 17504044). In certain embodiments, the content of N-2 is 0.5-2% (v/v), e.g., 0.5-1% (v/v), such as about 1% (v/v). In certain embodiments, the content of B-27 is 1-5% (v/v), e.g., 1-2% (v/v), such as about 2% (v/v).

In certain embodiments, the basal medium is a mixture of KnockOut DMEM, KnockOut DMEM/F 12, DMEM, DMEM/F 12, neurobasal medium, and supplemented with N-2 and B-27.

In certain embodiments, the medium has one or more of the following characteristics:
(i) the content of the serum substitute is 0.1-20% (v/v), e.g., 0.1-10%;
(ii) the content of Activin A is 1-100ng/mL, e.g., 1-50ng/mL;
(iii) the content of bFGF is 1-100ng/mL, e.g., 1-50ng/mL;
(iv) the content of the Wnt/β-catenin signal transduction inhibitor is 1-50 µM, e.g., 1-25 µM.

In certain embodiments, the medium has one or more of the following characteristics:
(i) the serum substitute is selected from: KnockOut^{™} SR (abbreviated as KSR), N-2, B-27, Physiologix^{™} XF SR, StemSure^{™} serum substitute supplement, or any combination thereof;
(ii) the Wnt/β-catenin signal transduction inhibitor is selected from IWP-2, XAV939, Wnt-C59 (C59), IWP-L6, IWR-1-endo, ICG-001, KY1220, or iCRT14.

In certain embodiments, the medium consists essentially of a basal medium, a serum substitute (e.g., KSR), Activin A, bFGF, a Wnt/β-catenin signal transduction inhibitor, the basal medium is a mixture of KnockOut DMEM, KnockOut DMEM/F12, DMEM, DMEM/F12, neurobasal medium, and supplemented with N-2 and B-27.

In certain embodiments, the medium comprises: 0.5-2% (v/v) of N-2, 1-5% (v/v) of B-27, 0.1-20% (v/v) of serum substitute (e.g., KSR), 1-100ng/mL Activin A, 1-100ng/mL bFGF, 1-50µM Wnt/β-catenin signal transduction inhibitor.

### Method of preparation

The third aspect of the present application provides a method for producing the pluripotent stem cells described in the first aspect or the cell population described in the second aspect, which comprises inhibiting the epithelium-to-mesenchymal transition of the pluripotent stem cells that are about to undergo gastrulation, the pluripotent stem cells are derived from or selected from the following cells: embryonic stem cells (ESC), induced pluripotent stem cells (iPSC) or epiblast stem cells (EpiSC), epiblast cells, blastocyst inner cell mass, pluripotent stem cells induced from somatic cells, somatic cells, somatic stem cells, embryos from post-implantation to pre- and post-gastrulation.

The fourth aspect of the present application provides a method for producing the pluripotent stem cells described in the first aspect or the cell population described in the second aspect, which comprises three-dimensional culture carried out to the pluripotent stem cells, the pluripotent stem cells are derived from or selected from: embryonic stem cells (ESC), induced pluripotent stem cells (iPSC) or epiblast stem cells (EpiSC), epiblast cells, blastocyst inner cell mass, pluripotent stem cells induced from somatic cells, somatic cells, somatic stem cells, embryos from post-implantation to pre- and post-gastrulation.

In certain embodiments, the method described in the third aspect or the fourth aspect comprises three-dimensional culture carried out to the pluripotent stem cell in the formation medium, the formation medium is selected from a basal medium supplemented with the following substances: a serum substitute, Activin A, bFGF, and a Wnt/β-catenin signal transduction inhibitor.

As used herein, the term "three-dimensional culture" refers to placing cells in conditions compatible with cell growth while allowing the cells to grow in more than one layer. Three-dimensional culture conditions typically include a scaffold of culture material that provides a three-dimensional structure to serve as a template for growth. The material forming the three-dimensional scaffold should be a biocompatible material.

A variety of different materials can be used to form a three-dimensional scaffold, non-limiting examples of which include fiberglass, polyethylenes, polypropylenes, polyamides (e.g., nylon), polyesters (e.g., Dacron), polystyrenes, polyacrylates, polyvinyl compounds (such as polyvinyl chloride; PVC), polycarbonates, polytetrafluoroethylene (PTFE; TEFLON), thermanox (TPX), nitrocellulose, polysaccharides (e.g., cellulose, chitosan, agarose), polypeptides (e.g., silk, gelatin, collagen), polyglycolic acid (PGA) and dextran.

In some embodiments, the three-dimensional scaffold can be made of materials that degrade over time under conditions of use, non-limiting examples of which include, polylactide, polyglycolide, poly(trimethylene carbonate), poly(lactic acid/glycolic acid) copolymer (poly(lactide-co-glycolide), i.e., PLGA), polyethylene terephthalate (PET), polycaprolactone, gut suture material, collagen (e.g., horse collagen foam), polylactic acid or hyaluronic acid, etc.

In other embodiments, the three-dimensional scaffold can also be constructed of non-biodegradable materials. A non-biodegradable material refers to a material that does not significantly degrade or decompose under the conditions in the medium. As non-limiting examples, exemplary non-biodegradable materials include nylon, Dacron, polystyrene, polyacrylates, polyethylene, polytetrafluoroethylene (PTFE), expanded polytetrafluoroethylene (ePTFE), and cellulose, etc.

In certain embodiments, the formation medium for producing pluripotent stem cells of the present application has one or more of the following characteristics:
(i) the serum substitute is selected from KnockOut^{™} SR (abbreviated as KSR), N-2, B-27, Physiologix^{™} XF SR, StemSure^{™} Serum Substitute Supplement or any combination thereof;
(ii) The Wnt/β-catenin signal transduction inhibitor is selected from IWP-2, XAV939, Wnt-C59 (C59), IWP-L6, IWR-1-endo, ICG-001, KY1220, iCRT14.

In certain embodiments, the basal medium included in the formation medium is selected from KnockOut DMEM, KnockOut DMEM/F12, DMEM, DMEM/F12, neurobasal medium, and any combination thereof.

In certain embodiments, the basal medium is selected from basal medium supplemented with N-2 (e.g., Gibco: Cat. No. 17502048) and B-27 (e.g., Gibco: Cat. No. 17504044). In certain embodiments, the content of N-2 is 0.5-2% (v/v), e.g., 0.5-1% (v/v), such as about 1% (v/v). In certain embodiments, the content of B-27 is 1-5% (v/v), e.g., 1-2% (v/v), such as about 2% (v/v).

In certain embodiments, the basal medium is KnockOut DMEM, KnockOut DMEM/F12, DMEM, DMEM/F12, neurobasal medium, and any combination thereof, and supplemented with N-2 and B-27.

In certain embodiments, the formation medium has one or more of the following characteristics:
(i) the content of the serum substitute is 0.1-20% (v/v), e.g., 0.1-15%;
(ii) the content of Activin A is 1-100ng/mL, e.g., 1-50ng/mL;
(iii) the content of bFGF is 1-100ng/mL, e.g., 1-50ng/mL;
(iv) the content of the Wnt/β-catenin signal transduction inhibitor is 1-50 µM, e.g., 1-25 µM.

In certain embodiments, the formation medium consists essentially of the following components: basal medium, serum substitute (e.g., KSR), Activin A, bFGF, Wnt/β-catenin signal transduction inhibitor, the basal medium is KnockOut DMEM, KnockOut DMEM/F12, DMEM, DMEM/F12, neurobasal medium, and any combination thereof, and supplemented with N-2 and B-27.

In some embodiments, the formation medium comprises: 0.5-2% (v/v) of N-2, 1-5% (v/v) of B-27, 0.1-20% (v/v) of serum substitute (e.g., KSR), 1-100 ng/mL of Activin A, 1-100 ng/mL of bFGF, 1-50 µM of Wnt/β-catenin signal transduction inhibitor.

In certain embodiments, the three-dimensional culture condition comprises: culturing the pluripotent stem cells in the presence of a three-dimensional scaffold to obtain a three-dimensional culture.

In certain embodiments, the three-dimensional scaffold is a hydrogel. In certain embodiments, the hydrogel comprises extracellular matrix. In certain embodiments, the extracellular matrix comprises one or more of laminin, collagen, fibrin, hyaluronic acid, chitosan, etc.

In some embodiments, the three-dimensional culture comprises the following steps:
(1) mixing a single cell suspension of the pluripotent stem cells before and after gastrulation with the hydrogel to obtain a gel-like mixture embedded with the pluripotent stem cells before and after gastrulation;
(2) cultivating the gel-like mixture in step (1) in the forming medium described in any embodiments above to obtain a three-dimensional culture.

In certain embodiments, the single cell suspension in step (1) can be obtained from primary tissues, adherent cultured cells and aggregates by any mechanical or chemical means, e.g., physical force (mechanical dissociation, such as cell scraper, trituration by pinhole pipette, fine needle aspiration, vortex disaggregation, and forced filtration through fine nylon and stainless steel mesh screens), enzymes (enzymatic dissociation such as trypsin, collagenase, Acutase, etc.) or a combination of both of them.

In certain embodiments, the hydrogel in step (1) is an extracellular matrix comprising laminin and type IV collagen. In certain embodiments, the extracellular matrix further comprises nestin and heparan sulfate proteoglycans.

In certain embodiments, in step (1), the pluripotent stem cells are mixed with the hydrogel, and the mixture is spread on a culture container (e.g., a petri dish) and the incubation is performed.

In certain embodiments, the method further comprises: (3) isolation of cells from the three-dimensional culture, thereby obtaining the pluripotent stem cells. In certain embodiments, said isolation comprises mechanical isolation and/or enzymatic digestion. In certain embodiments, said isolation comprises mechanical isolation of cells from attachment to the extracellular matrix and formation of single cells by enzymatic digestion.

In certain embodiments, the method further includes passage of the pluripotent stem cells in step (3).

In certain embodiments, the passage includes inoculating the pluripotent stem cells obtained in step (3) in a passage medium for culturing, and the passage medium is selected from the formation medium described in any of the above embodiments.

In certain embodiments, the passage medium consists essentially of basal medium, serum substitute (e.g., KSR), Activin A, bFGF, Wnt/β-catenin signal transduction inhibitor, the basal medium is KnockOut DMEM, KnockOut DMEM/F12, DMEM, DMEM/F12, neurobasal medium and any combination thereof and supplemented with N-2 and B-27.

In certain embodiments, the passage medium comprises: 0.5-2% (v/v) of N-2, 1-5% (v/v) of B-27, 0.1-20% (v/v) of serum substitute (e.g., KSR), 1-100 ng/mL of Activin A, 1-100 ng/mL of bFGF, 1-50 µM of Wnt/β-catenin signal transduction inhibitor.

In certain embodiments, the passaging comprises:
(a) mixing the pluripotent stem cells obtained in step (3) with a hydrogel defined in step (1);
(b) mixing the passage medium with the hydrogel to obtain a gel-like carrier, the passage medium is as defined in any of the above embodiments; wherein, steps (a) and (b) are carried out simultaneously or in any order;
(c) spreading the liquid mixture described in step (a) on the gel-like carrier described in step (b) to obtain the gel-like mixture;
(d) culturing the gel-like mixture of step (c) in fresh passage medium.

In certain embodiments, said passaging optionally further comprises isolating cells from the gel-like mixture obtained in step (d).

In certain embodiments, the liquid mixture of the passage medium and the hydrogel is spread on a culture container (e.g., a petri dish) and the incubation is performed to obtain a culture container with the gel-like carrier attached to the surface.

In certain embodiments, the pluripotent stem cells described in the first aspect of the present application are prepared by the method described in the third or fourth aspect of the present application.

### Application

In the fifth aspect, the present application provides a pharmaceutical composition, which comprises the pluripotent stem cell described in the first aspect or the cell population described in the second aspect, and a pharmaceutically acceptable carrier and/or excipient.

In certain embodiments, the pharmaceutical composition may be in any form known in the medical art. For example, the pharmaceutical composition can be tablets, pills, suspensions, emulsions, solutions, gels, capsules, powders, granules, elixirs, lozenges, suppositories, injections (including injection solution, lyophilized powder) and other forms. In certain preferred embodiments, the pharmaceutical composition is an injection (including injection solution, lyophilized powder).

In certain embodiments, the pharmaceutical composition comprises a pharmaceutically acceptable sterile isotonic aqueous or non-aqueous solution (e.g., balanced salt solution or physiological saline), dispersion, suspension or emulsion. In certain embodiments, the pharmaceutical composition may be implanted in the form of a suspension, gel, colloid, slurry or mixture.

In a sixth aspect, the present application provides a method for in vitro production of an endoderm, ectoderm or mesoderm cell, or in vitro production of a tissue or an organ (e.g., a organoid), comprising: culturing the pluripotent stem cell described in the first aspect, the cell population described in the second aspect or the pharmaceutical composition described in the fifth aspect under a condition that allows differentiation (e.g. directional differentiation) of the pluripotent stem cell.

In another aspect, the present application also relates to the use of the pluripotent stem cell described in the first aspect, the cell population described in the second aspect, or the pharmaceutical composition described in the fifth aspect for producing an endoderm, ectoderm or mesoderm cell in vitro, or producing a tissue or an organ (e.g. a organoid) in vitro.

In certain embodiments, the cell is selected from the group consisting of a germ cell (e.g., a primordial germ cell), an epithelial cell, a connective tissue cell, a nerve cell (e.g., neuronal cell), an adipocyte, a pancreatic cell (e.g., insulin producing cell), a liver cell, a renal cell, a bone cell, a hematopoietic cell, an endothelial cell, a retinal cell, and a muscle cell (e.g., a cardiomyocyte).

In another aspect, the present application also relates to the pluripotent stem cell described in the first aspect, the cell population described in the second aspect, or the pharmaceutical composition described in the fifth aspect, or a tissue or an organ produced by them in vitro (e.g. a organoid) for being used as a disease model and/or a drug screening model.

In the seventh aspect, the present application provides a method for the regeneration or repair of a tissue or an organ, or for transplantation of a cell, a tissue or an organ, comprising: using the pluripotent stem cell described in the first aspect, the cell population described in the second aspect, or the pharmaceutical composition described in the fifth aspect.

In some embodiments, the method comprises: administering (e.g., transplanting) the pluripotent stem cell described in the first aspect, the cell population described in the second aspect, or the pharmaceutical composition described in the fifth aspect to a subject in need thereof.

In some embodiments, the method comprises: inducing in vitro the differentiation and/or proliferation of the pluripotent stem cell described in the first aspect and the cell population described in the second aspect, and administering (e.g., transplanting) the differentiated or expanded said pluripotent stem cell or cell population (e.g., a organoid), or a pharmaceutical composition comprising said differentiated or expanded said pluripotent stem cell or cell population (e.g., a organoid) to a subject in need thereof.

In another aspect, the present application also relates to the use of the pluripotent stem cell described in the first aspect, the cell population described in the second aspect, or the pharmaceutical composition described in the fifth aspect for (i) regeneration or repair of a tissue or an organ, or (ii) transplantation of a cell, a tissue or an organ, or in the preparation of a medicament for (i) regeneration or repair of a tissue or an organ, or (ii) transplantation of a cell, a tissue or an organ.

### Definition of terms

In the present application, unless otherwise specified, the scientific and technical terms used herein have the meanings commonly understood by those skilled in the art. Moreover, the laboratory operation steps used herein in the fields of stem cells, biochemistry, nucleic acid chemistry, immunology, etc. are all routine steps widely used in the corresponding fields. Meanwhile, in order to better understand the present application, definitions and explanations of related terms are provided below.

As used herein, the term "naive" refers to the naive state of mammalian embryonic stem cells, in which ESCs need to escape from the naive state before they can respond to the induction signal of lineage differentiation. The term "primed" refers to the primed state of mammalian embryonic pluripotent stem cells, the fate of which has been partially determined, and has low pluripotency and differentiation potential.

As used herein, the term "fPSC" refers to a formative pluripotent stem cell. In some embodiments, the pluripotent stem cell of the present application (e.g., the pluripotent stem cells obtained in Example 1) are referred to as fPSC.

As used herein, the term "pluripotent stem cell" refers to a stem cell having pluripotency and proliferative potential, the pluripotency is the ability of stem cells to differentiate into all cells derived from the ectoderm, mesoderm and endoderm cells as well as germ cells present in living organisms. Examples of currently known pluripotent stem cell include, but are not limited to, an embryonic stem cell (ESC), an induced pluripotent stem cell (iPSC), and an epiblast stem cell (EpiSC).

As used herein, the term "EX" refers to the number of days of embryonic development, counted from the time when sperm and egg combine to form a single-cell embryo, for example, E5.5 refers to day 5.5 of embryonic development.

As used herein, the term "self-renewable" refers to cells capable of self-renewal over multiple passages without significant changes in cell properties. In some embodiments, the number of passages is at least about 5, at least about 10, at least about 20, at least about 30, at least about 50, or at least about 100.

As used herein, the term "differentiation" means to make an undifferentiated cell into a differentiated cell, and in practice it means that an undifferentiated cell loses its original ability to differentiate into a specific cell type and/or become a specific cell lineage. The pluripotent stem cells of the present application can be used not only to generate terminally differentiated cells that have been irreversibly differentiated into specific cell types, but can also be used to generate partially differentiated cells capable of becoming various types of cells. These cells include germ cells and precursor cells of the three germ layers: endoderm, mesoderm and ectoderm. Undifferentiated cells and differentiated cells can be identified by the expression of specific marker genes. For example, stem cells can be identified by the expression of the marker genes OCT3/4 and NANOG. For example, differentiated cells can be determined by the expression of the following marker genes: PAX6, SOX1, and ZIC1 for ectoderm; NOX1 for mesoderm; BRACHYURY (BRY; also denoted by a "T") for mesendoderm; SOX17, CXCR4 and FOXA2 are specifically for endoderm. The term "directed differentiation" refers to the induction of differentiation into specific cell types by culture conditions.

As used herein, the term "expansion" or "proliferation" refers to maintaining cells substantially without differentiation and ultimately cell growth, i.e., increasing the cell population (e.g., to at least 2-fold) without concomitant increased differentiation .

As used herein, the term "in vitro" refers to an artificial environment, and the processes and reactions therein. In vitro environments are exemplified by, but not limited to, test tubes and cell cultures.

As used herein, the term "in vivo" refers to the natural environment (i.e., an animal or a cell) and the processes and reactions within it.

As used herein, the term "basal medium" refers to any medium capable of supporting cell growth, generally comprising inorganic salts, vitamins, glucose, buffer systems and essential amino acids, and generally having an osmotic pressure of about 280-330 mOsmol.

As used herein, the term "serum substitute" has the meaning known to those skilled in the art, which refers to the composition or formulation used as a serum substitute in the process of culturing pluripotent stem cells while maintaining an undifferentiated state. That is, serum substitute is capable of supporting the growth of undifferentiated pluripotent stem cells without supplementation of serum. In certain exemplary embodiments, the serum substitute comprises: one or more amino acids, one or more vitamins, one or more trace metal elements. In some cases, the serum substitute may further comprises one or more components selected from the group consisting of: albumin, reduced glutathione, transferrin, insulin, etc. Non-limiting examples of the serum substitute includes, but are not limited to, KnockOut^{™} SR (abbreviated as KSR), N-2, B-27, Physiologix^{™} XF SR, StemSure^{™} serum substitute supplement, etc.

As used herein, the term "pharmaceutically acceptable carrier or excipient" refers to a carrier and/or excipient that is pharmacologically and/or physiologically compatible with the subject and the active ingredient, which are well known in the art (see, e.g., Remington's Pharmaceutical Sciences. Edited by Gennaro AR, 19th ed. Pennsylvania: Mack Publishing Company, 1995), and includes, but is not limited to: a pH adjusting agent, a surfactant, an ionic strength enhancer, an agent to maintain osmotic pressure, an agent to delay absorption, a diluent, an adjuvant, a preservative, etc. For example, pH adjusting agents include, but are not limited to, phosphate buffers. Surfactants include but are not limited to cationic, anionic or nonionic surfactants e.g., Tween-80. Ionic strength enhancers include, but are not limited to, sodium chloride. Agents to maintain osmotic pressure include, but are not limited to, sugars, NaCl, and analogs thereof. Agents that delay absorption include, but are not limited to, monostearates and gelatin. Diluents include, but are not limited to, water, aqueous buffers (e.g., buffered saline), alcohols and polyols (e.g., glycerol), etc. Adjuvants include, but are not limited to, aluminum adjuvants (e.g., aluminum hydroxide), Freund's adjuvant (e.g., complete Freund's adjuvant), etc. Preservatives include, but are not limited to, various antibacterial and antifungal agents, such as thimerosal, 2-phenoxyethanol, parabens, chlorobutanol, phenol, sorbic acid, etc. In certain embodiments, the pharmaceutically acceptable carrier or excipient is a sterile isotonic aqueous or non-aqueous solution (e.g., balanced salt solution or physiological saline), dispersion, suspension or emulsion.

As used herein, the term "about" refers to a value or composition within an acceptable error range for a particular value or composition as determined by those ordinary skilled in the art, which will partially depend on how the value or composition is measured or determined, that is, the limitation of the measurement system. For example, when "about" is used to describe a measurable value (e.g., concentration of a substance, mass ratio, etc.), it means including a range of ±10%, ±5%, or ±1% of a given value.

### Beneficial effects of the application

The present application obtains stable formative pluripotent stem cells (fPSCs) in vitro for the first time. Compared with embryonic stem cells or other pluripotent stem cells, fPSCs have obvious advantages: the pluripotent stem cells of the present application have the potential to differentiate into all embryonic cells, for example, they can efficiently, rapidly differentiate into germ cells or germ precursor cells or germ-like precursor cells, and cells of endodermal, mesodermal, ectodermal lineages; compared with naive embryonic stem cells, the pluripotent stem cells of the present application have faster response speed and differentiation efficiency to differentiation signals, and more homogeneous when differentiated into other cells. In addition, the pluripotent stem cells of the present application can maintain stability in vitro for a long time. Therefore, the pluripotent stem cells of the present application have important application value in stem cell differentiation, organoids, cell transplantation, tissue or organ repair or regeneration, and the like.

Embodiments of the present application will be described in detail below with reference to the drawings and examples, but those skilled in the art will understand that the following drawings and examples are only for illustrating the present application, rather than limiting the scope of the present application. Various objects and advantages of the present application will become apparent to those skilled in the art according to the accompanying drawings and the following detailed description of the preferred embodiments.

### BRIEF DESCRIPTION OF THE DRAWINGS

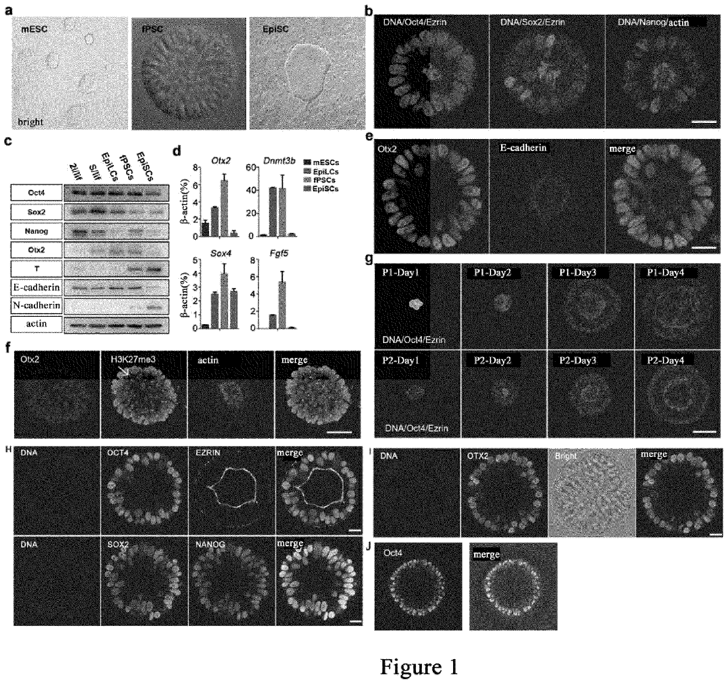

Figures 1A-1J show the identification results of the basic characteristics of fPSCs in Example 2. (A) Morphology of mouse naive embryonic stem cells (mESCs, the figure shows 2i/lif is the mESCs cultured under the condition of 2i/lif), mouse formative pluripotent stem cell line (fPSCs, in the figure it is shown as mfPSC) and epiblast stem cells (EpiSCs). (B) Immunofluorescent co-staining results of pluripotent stem cell markers OCT4, SOX2, and NANOG with polar molecular marker EZRIN or cytoskeletal molecule ACTIN; pluripotent factor OCT4 is highly expressed in fPSCs, SOX2 and NANOG is expressed in a mosaic style. (C) Western blotting shows the expression of various marker molecules in mESCs (two conditions of 2i/Lif and serum S/Lif), epiblast-like cells (EpiLCs, a transient epiblast-like cell), fPSCs and EpiSCs. (D) Quantitative RT-PCR results show comparison of mRNA expression levels of putative formative markers in mESCs, fPSCs, and EpiSCs; fPSCs highly express the formative pluripotent markers of OTX2, DNMT3B, SOX4, and FGF5 relative to mESCs, EpiLCs, and EpiSCs. (E) Immunofluorescence results of co-staining of formative markers OTX2 and Cadherin in fPSCs; it shows that fPSCs highly express formative pluripotent factor OTX2 and E-cadherin. (F) H3K27me3 immunofluorescence results showed that one of the two chromosomes in female fPSCs was inactivated. (G) An fPSCs cell line in which OCT4 distal enhancer drives GFP expression shows that, unlike mESCs where OCT4 utilizes a distal enhancer, OCT4 in fPSCs utilizes a proximal enhancer. (H) Immunofluorescence staining results of pluripotent factors OCT4, SOX2 and NANOG and polar molecule EZRIN in human formative pluripotent stem cells (hfPSCs); it shows that hfPSCs highly express pluripotent factors OCT4, SOX2 and NANOG, and staining results of EZRIN show that hfPSC has a polar rosette structure. (I) Immunofluorescent staining results of the expression of the formative marker OTX2 in hfPSCs shows that hfPSCs highly express the formative pluripotent factor OTX2. Figure 1J shows the pluripotent marker OCT4 staining results of fPSCs established from the inner cell mass extracted from mouse E3.5 blastocysts, showing that the fPSCs established from the inner cell mass extracted from mouse E3.5 blastocysts have a rosette structure.

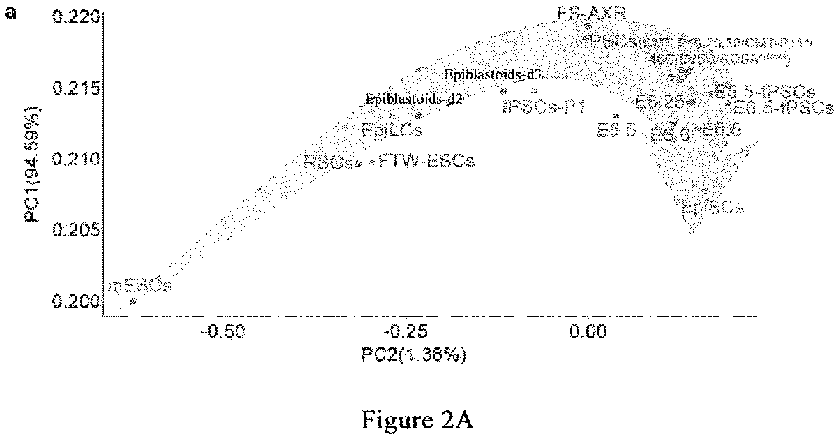

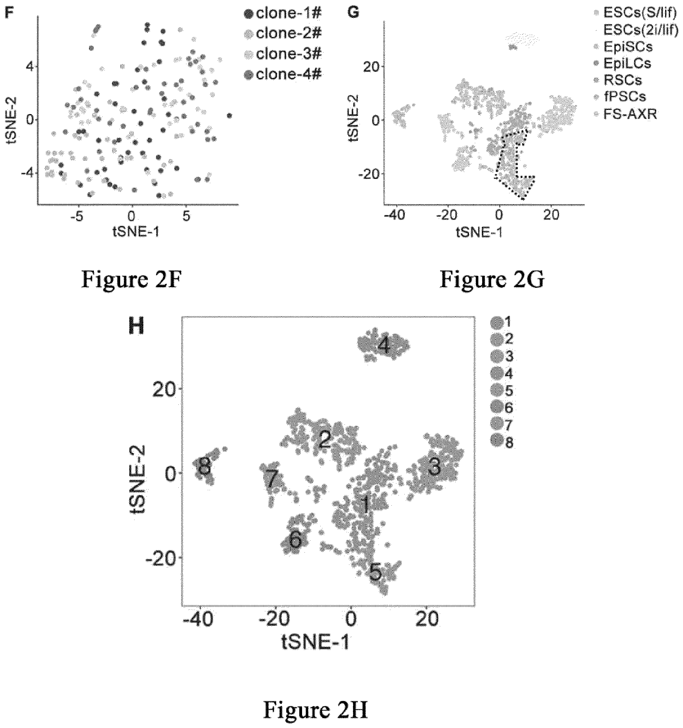

Figures 2A-2H show the results of genome-level characteristic analysis of mouse fPSCs in Example 3. (A) PCA analysis reveals that the genome-wide expression of fPSCs (shown as E5.5-fPSCs and E6.5-fPSCs in the figure) was similar to that of E6.0-E6.5 epiblast cells in vivo, but significantly different from mESCs and EpiSCs. (B) Genome-wide spatiotemporal transcriptome analysis reveals that fPSCs are most similar to E6.0 mouse epiblast cells and part of E6.5 mouse epiblast cells. (C) Differential gene analysis reveals that fPSCs specifically highly express 535 genes, including previously predicted formative genes and many genes that have important functions in ESCs leaving the native state, and these genes are marker molecules of fPSCs. (D) Quantitative RT-PCR verification of marker molecules of fPSCs, ZIC2, ZIC5, LIN28B, ESRP1, UTF1, ETV1, ETV4, GRHL2, CLDN6, CLDN7 are highly expressed in fPSCs, these are marker molecules of fPSCs. (E) Compared with naive mESCs, formative genes MIXL1, EOMES, TCFL5, and WNT3 are highly expressed in mouse fPSCs. The values are transcriptome FPKM values. (F-H) shows the results of single-cell sequencing analysis of mouse fPSCs and the results of integrated analysis with single-cell sequencing data of various other types of pluripotent cells. Figure F shows that fPSCs single cells derived from 4 independent clones (shown as clone-1#, clone-2#, clone-3#, clone-4# in the figure) are relatively homogenous at the transcriptome level, with no obvious grouping phenomenon. In Figure (G), the combined analysis of single-cell sequencing results of multiple types of pluripotent cells further confirmed that naive ESCs had no obvious grouping phenomenon at the single-cell level, while primed EpiSCs were divided into two subpopulations, which is consistent with the reported results; FS cells were clearly divided into 3 cell subpopulations, namely subpopulations 3, 6 and 7 in Figure H, compared to fPSCs without obvious grouping. fPSC grouping is marked by dashed lines. log2FPKM refers to the base 2 logarithm value after normalizing the gene expression value FPKM.

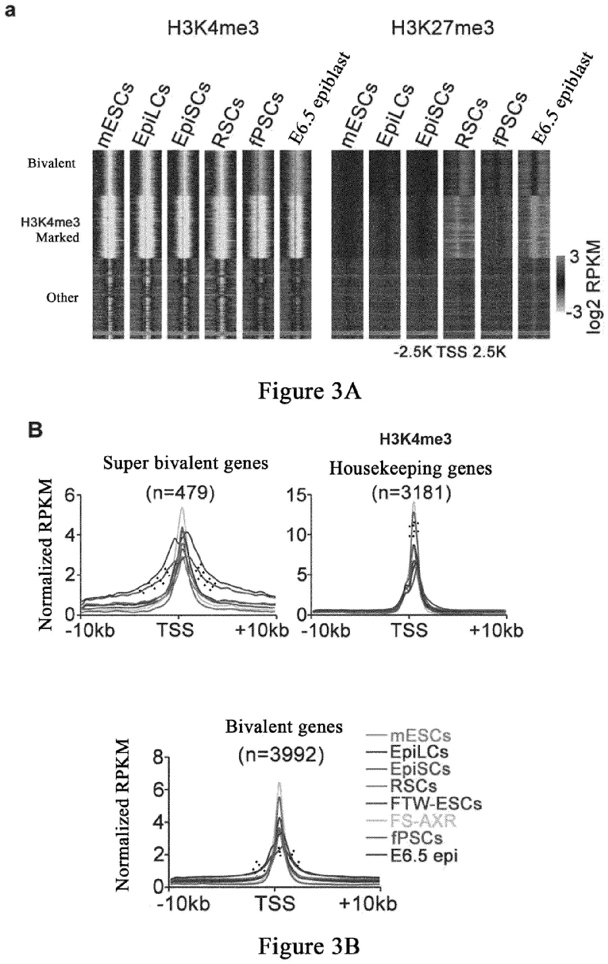

Figures 3A-3J show the results of characteristic analysis of mouse fPSCs in Example 4 at the epigenetic level. (A) Distribution of H3K4me3 and H3K27me3 epigenetic modifications on promoters (±2.5Kb) at the genome-wide level in various pluripotent cells, showing that the epigenetic modifications in the promoter region of fPSCs are similar to those of E6.5 epiblast cells (shown as E6.5 epi in the figure) isolated in vivo. (B) Distribution of H3K4me3 on promoters (20Kb of upstream or downstream, shown as -10kb and +10kb in the figure) at the genome-wide level in super bivalent genes, housekeeping genes and bivalent genes, showing that fPSCs were most similar to E6.5 embryonic epiblast cells isolated in vivo in super bivalent genes. Wherein fPSCs are marked by a dotted line. (C) super bivalent genes identified in all stem cells, showing that the number of super bivalent genes in fPSCs (n = 329) is significantly higher than that of other in vitro stem cell lines (n = 68-197), which is the closest to the in vivo E6.5 epiblast cells (n=440). (E-F) Comparison of methylation levels at the genome-wide level, the results show that compared with other cells, fPSCs are closest to the in vivo E6.5 epiblast cells (shown as E6.5 epi in the Figure) (the overall discrimination is limited, and probably no good comparison method has yet been found, or DNA methylation itself is not necessarily a good way to compare these cells). (G) The epigenetic modification of H3K4me3 and H3K27me3 at the position of formative gene OXT2 and ZIC5 shows that fPSCs are closer to E6.5 epiblast cells. (H) Epigenetic modification of three germ layer cell lineage marker molecule (mostly super bivalent genes), especially H3K3me3 in the basic presence or absence relationship in the promoter region (continuous strong H3K4me3 signal) shows that fPSCs are closer to E6.5 epiblast cells. (I) DNA methylation and H3K4me3/H3K27me3 enrichment at the promoter of super bivalent/covalent modification genes (UTF1, FGF5, ZFP13, ZSCAN10, ZIC2, ESRP1, T, GATA6, LHX5, HAND1, ISL1, NKX2-5, NR4A2, PDX1) in mESC, EpiLC, EpiSC, fPSC and E6.5 epiblast cells, the results show that compared with other cells, fPSCs are closest to in vivo E6.5 epiblast cells. (J) Super bivalent gene list of fPSCs. log2RPKM refers to the base 2 logarithm value after normalizing the gene expression value RPKM; TSS refers to the transcription start site; HK.ave refers to the average normalized RPKM value of the housekeeping gene; R refers to the correlation coefficient; mCG/CG refers to the ratio of methylated level to unmethylated level in genome.

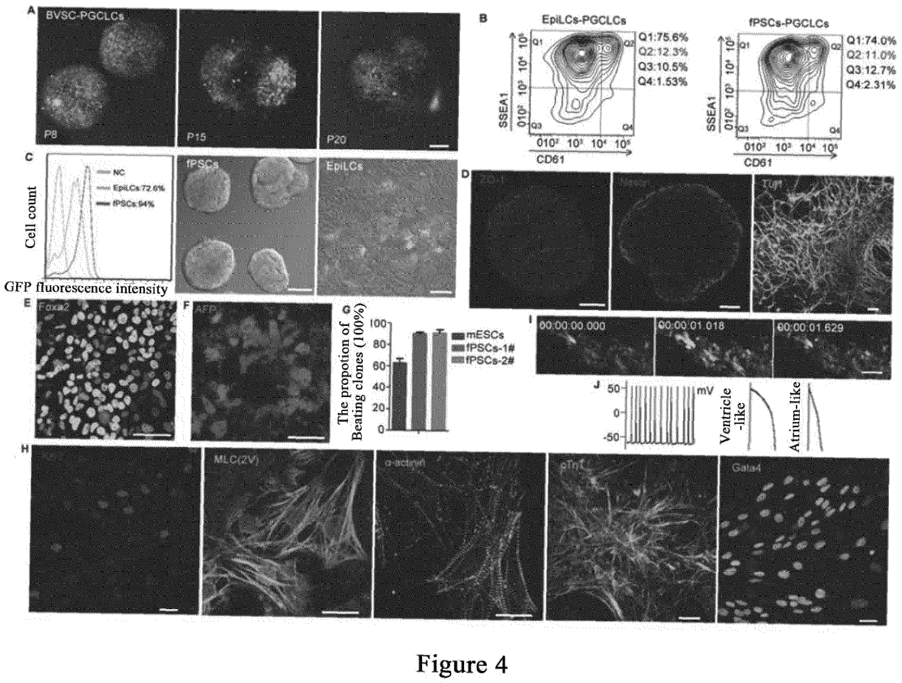

Figure 4A-4J show the detection results of in vitro differentiation of mouse fPSCs in Example 5. (A) Results of differentiation of fPSCs into primordial germ cell-like cell (PGCLCs) at 8th(P8), 15th, and 20th passages, BV (green) and SC (blue) showed successful differentiation. (B) The differentiation efficiency of fPSCs into potentially functional PGCLCs (SSEA1 and CD61 double positive cells) was 11%, which was close to that of the unstable epiblast-like cells (EpiLCs) with the best known differentiation efficiency (12.3%). (C) The differentiation efficiency of fPSCs to neural precursor cells (SOX1 positive) (indicated by GFP fluorescence in the Figure) was as high as 94% on the second day, which was significantly higher than 72.6% of EpiLCs; and compared with the ESCs monolayer differentiation system (68%), not only the efficiency is high, but also the time is much shorter (5-6 days), and it is more synchronized. (D) After 2 days of differentiation, the neural precursor cells derived from fPSCs can form neuron-like cells (multiple nerve cell marker molecules ZO1, Nestin, Tuj 1). (E) The differentiation efficiency of fPSCs to endoderm precursor cells (FOXA2) reached 88%. (F) The endodermal precursor cells derived from fPSCs can differentiate into liver-like cells after 7 days (AFP is a liver cell marker). (G) The differentiation efficiency of fPSCs to mesodermal cells (the proportion of beating cardiomyocyte like cells, i.e., the proportion of beating clones) reached 90%, which was significantly higher than that of ESCs differentiated by commonly used EB (~60%). (H) Cardiomyocyte-like cells derived from fPSCs express proliferation marker Ki67, and simultaneously express cardiomyocyte markers (MLC, α-actinin, cTnT and gATA4); in addition, sarcomeres are clearly visible. (I-J) After 3-4 days of maturation of fPSCs-derived cardiomyocyte-like cells, calcium ion release (I) and electrophysiological activity (J) can be detected; at the same time, action potentials (AP), typical ventricle-like traces and atrium-like AP were observed in the cardiomyocyte-like cells differentiated from fPSCs after 7-8 days of differentiation.

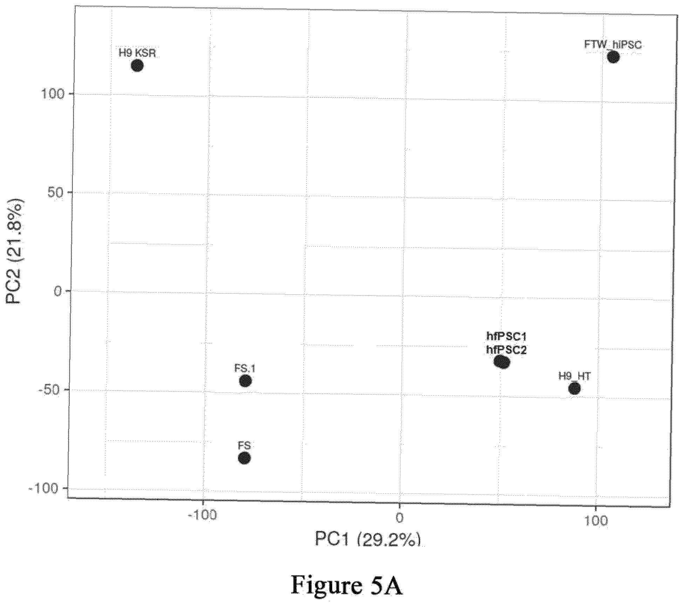

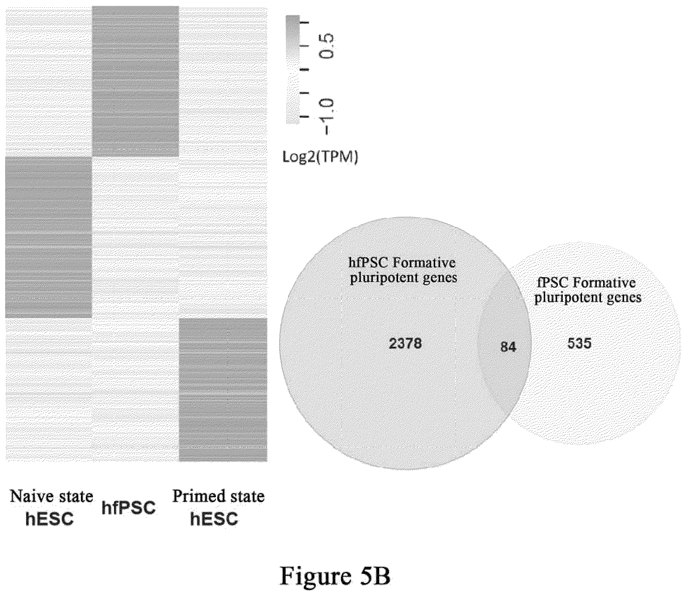

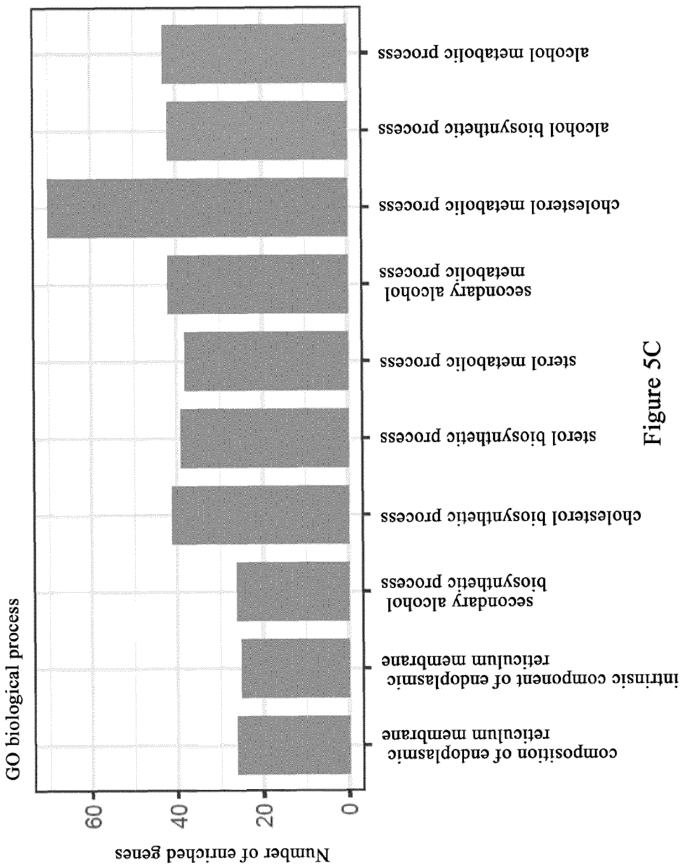

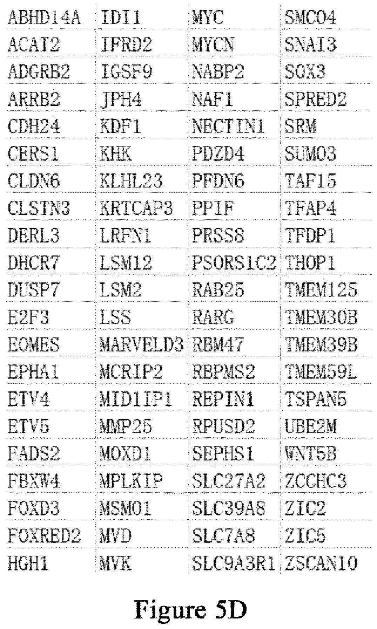

Figures 5A-5D. Figure 5A shows that the genome-wide expression pattern of hfPSCs is between H9_HT (original state hESC (naive hESC) and H9 KSR (primed hESC)), the pluripotent state is an activated state (a formative state), and is quite different from FS (Smith et al., 2020) and FTW_hiPSC (Wu et al., 2020). Figure 5B shows that hfPSCs specifically highly expressed genes; there are 84 formative genes in the intersection of hfPSCs and fPSCs, and these genes are conserved marker molecules shared by fPSCs and hfPSCs. Figure 5C shows that the high level of cholesterol, sterol metabolism and fat metabolism of hfPSCs is unique to the activated state (the formative state) through cluster analysis of biological processes of genes highly expressed in hfPSCs. Figure 5D shows a list of 84 formative genes in the intersection of hfPSC formative genes and fPSC formative genes, and these genes are conserved marker molecules shared by fPSCs and hfPSCs. log2TPM refers to the base 2 logarithm value after normalizing the gene expression value RPKM.

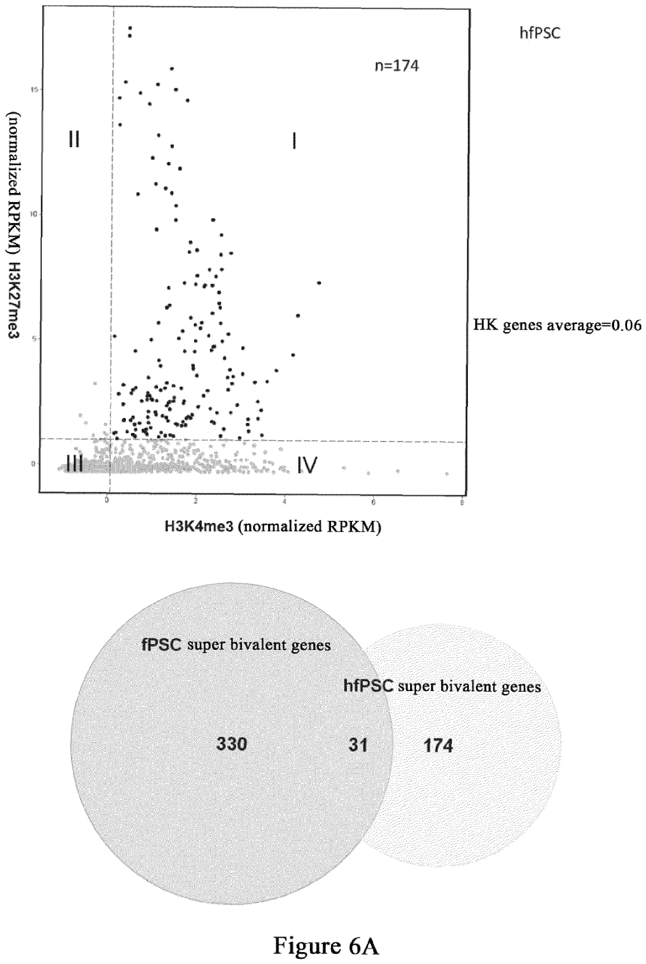

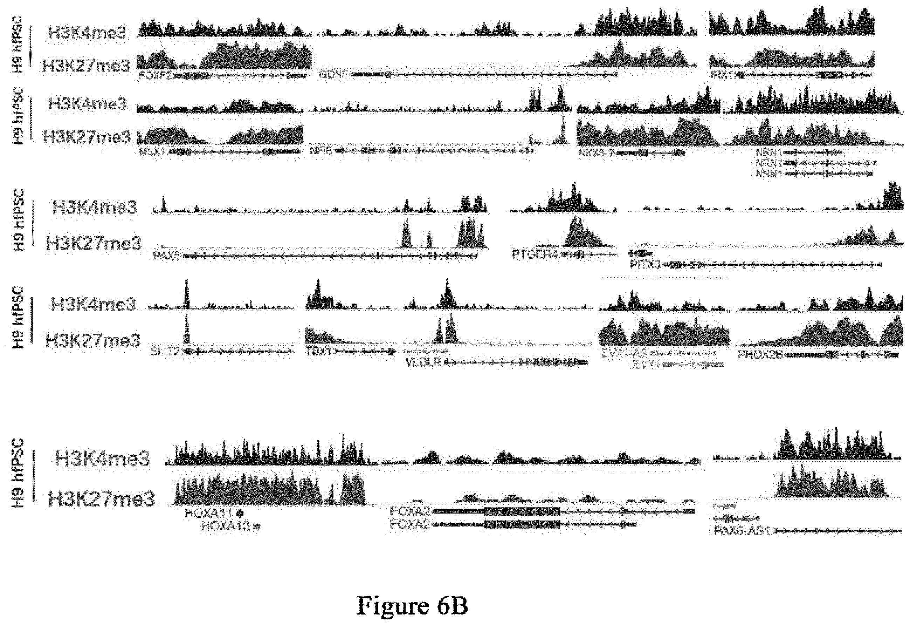

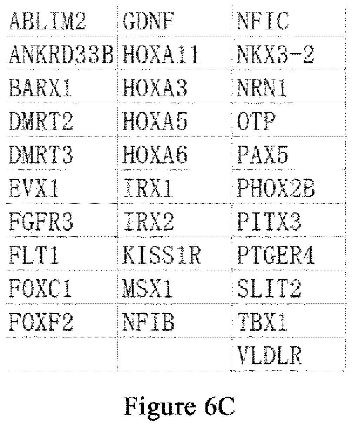

Figure 6A-6C shows the number of super bivalent genes (n=174) identified in hfPSC (shown as 3D_hfPSC in the figure), where all genes in quadrant I are super bivalent genes, and the genes in quadrants II, III and IV are non-super bivalent genes; there are 31 super bivalent genes in the intersection of hfPSC super bivalent genes and fPSC super bivalent genes. Figure 6B shows H3K4me3 and H3K27me3 super covalent modification patterns of H3K4me3 and H3K27me3 at the promoter regions of HOXA cluster, FOXA2, PAX6, FOXF2, GDNF, IRX1, MSX1, NFIB, NKX3-2, NRN1, PAX5, PTGER4, PITX3, SLIT2, TBX1, VLDLR, EVX1, PHOX2B genes in hfpscs (H9hfpscs). Figure 6C shows a list of 31 super bivalent genes shared by the intersection of hfPSCs and fPSCs.

### DETAIL DESCRIPTION OF THE PRESENT APPLICATION

The present application will now be described with reference to the following examples, which are intended to illustrate the present application, (but not to limit it).

Unless otherwise specified, the molecular biology experiment methods and immunoassay methods used in the present application are basically with reference to the methods described in J. Sambrook et al., Molecular Cloning: A Laboratory Manual, 2nd Edition, Cold Spring Harbor Laboratory Press, 1989, and F. M. Ausubel et al., Short Protocols in Molecular Biology, 3rd Edition, John Wiley & Sons, Inc., 1995; the use of restriction endonucleases is in accordance with the conditions recommended by the product manufacturer. Those skilled in the art understand that the Examples describe the present application by way of example and are not intended to limit the scope of the present application.

The source of the main reagent involved in the following examples is as follows:
1. Reagents for cell culture of mESCs, iPSC and EpiSCs:
   Feeder-free dish (Millipore, #901771), N2B27 medium with MPD035901 (LC Laboratories, #P-9688), Chir99021 (LC Laboratories, #C-6556), hLIF (human leukemia inhibitory factor, Millipore, #ESG1107). Mitomycin C (Sigma-Aldrich, #M0503), KSR (Gibco, #10828028), Activin A (PeproTech, #100-18B), bFGF (R&D Systems, #233-FB), XAV939 (Sigma-Aldrich, #X3004). Collagenase IV (Gibco, #17104-019), trypsin (Gibco, #12605010) Y-27632 (TOCRIS, #1254).
2. Reagents for immunofluorescence staining analysis:
   Paraformaldehyde (PFA) (Sigma-Aldrich, #158127), Triton X-100 (Amresco, #0694), BSA (Sigma-Aldrich, #A4378), Hoechst 33342 (Invitrogen, #H3570). Primary antibodies included: rabbit anti-Ezrin (Abcam, #Ab76247), mouse anti-GM130 (BD Biosciences, #BD610822), rat anti-Nestin (Millipore, #MAB353), rabbit anti-Zo-1 (Abcam, #Ab214228), rabbit anti-Tuj 1 (Sigma-Aldrich, #T2200), goat anti-Oct4 (Santa Cruz Biotechnology, #sc-8628), rabbit anti-Nanog (Abcam;, #ab80892), mouse anti-Sox2 (CST; #4900S), rabbit anti-Ki-67 (Abcam, #ab15580), mouse anti-cardiac troponin T (cTnT, Thermo Fisher Scientific, #MS-295-P0), mouse anti-α-actin (Sigma-Aldrich, #A7732), rabbit anti-myosin light chain (MLC) 2v (ProteinTech Group, #10906-1-AP), rabbit anti-Foxa2 (CST, #8186S), goat anti-Gata6 (R&D systems, #AF1700), goat anti-Otx2 (R&D systems, #AF1979), goat anti-Gata4 (Santa Cruz Biotechnology, #sc-1237), rabbit anti-E-cadherin (Santa Cruz Biotechnology, #sc-7870), mouse anti-α-SMA (Santa Cruz Biotechnology, #sc-53142), mouse anti-GFAP (Sigma-Aldrich, #G3893), mouse anti-AFP (CST, #3903S). Secondary antibodies were from Jackson ImmunoReseach and included: 488 AffiniPure donkey anti-rabbit IgG (H+L) (#711-545-152), AlexaFluor^{®}594 AffiniPure donkey anti-rabbit IgG (H+L) (#711-585-152), Cy^{™}5 AffiniPure donkey anti-rabbit IgG (H+L) (#711-175-152), AlexaFluor^{®}488 AffiniPure donkey anti-mouse IgG (H+L) (#711-545-150), AlexaFluor^{®}647 AffiniPure donkey anti-mouse IgG (H+L) (#715-605-151), AlexaFluor^{®}594 AffiniPure donkey anti-mouse IgG (H+L) (#715-585-150), AlexaFluor^{®}488 AffiniPure donkey anti-goat IgG (H+L) (#705-545-003), AlexaFluor^{®}488-AffiniPure donkey anti-mouse IgG (H+L) (#712-545-153).
3. Reagents used in quantitative RT-PCR:
   RNAzol (Mrcgene, #RN190), PrimeScript RT Reagent Kit (TaKaRa, #RR037A), EvaGreen 2qPCR MasterMix (ABM, MasterMix-S).
4. Reagents for western blot analysis:
   SuperSignalTM West Femto Maximum Sensitivity Substrate (Thermo Fisher Scientific, #34095). Primary antibodies used included: mouse anti-β-actin (Yeasen, #30101ES50), goat anti-Oct4 (Santa Cruz Biotechnology, #sc-8628), mouse anti-Sox2 (CST, #4900S), rabbit anti-Nanog (Abcam, #ab80892), goat anti-T (CST, #81694), goat anti-Otx2 (R&D Systems, #AF1979), rabbit anti-Foxa2 (CST, #8186S), rabbit anti-E-cadherin (Santa Cruz Biotechnology, #sc-7870), mouse anti-N-cadherin (BD, #610920). Secondary antibodies were from Jackson ImmunoResearch and included: peroxidase AffiniPure goat anti-rabbit IgG (#111-035-003), peroxidase AffiniPure goat anti-mouse IgG (#115-035-003), peroxidase and rabbit anti-goat IgG (#305-035-003).
5. Alkaline phosphatase staining:
   BCIP/NBT Alkaline Phosphatase Color Development Kit (Beyotime, #C3206).
6. Induction of PGCLC and three germ layers:
   G-MEM medium (1) (Gibco, #11710-035), sodium pyruvate (Gibco, #11360070), non-essential amino acids (Gibco, #11140050), b-mercaptoethanol (Invitrogen, #21985-023), L-glutamine (Gibco, #35050061), streptomycin (Gibco, #15140122), BMP4 (R&D Systems, #315-27), SCF (R&D Systems, #455-MC-010), BMP8a (R&D Systems , #1073-BP-010), EGF (R&D Systems, #2028-EG), RPMI medium 1640(1) (Gibco, #11875-093), VC (Vitamin C, Sigma-Aldrich, #A7506), B27 supplement minus insulin (Gibco, #A1895601), dexamethasone (Sigma-Aldrich, #D4902), HGF (PeproTech, #100-39), oncostatin M (R&D Systems, #295-OM).
7. Electrophysiology and intracellular Ca²⁺ measurement:
   Fluo-3AM (Beyotime, #S1056).
8. Chimera experiments of fPSC and culture of embryos after implantation:
   M2 medium (Sigma-Aldrich, #M7167), FBS (Millipore, #ES 009B), CMRL 1066 medium (Gibco, #11530037), sodium pyruvate (Invitrogen, #11360070), glutamine (Invitrogen #25030081), N2 (Invitrogen, #17502048), B27 (Invitrogen, #17504044).
9. Antibodies used in STAR ChIP-seq:
   H3K4me3 (in-house), H3K27me3 (Diagenode, pAb-069-050), rSAP (NEB, #M0371).

The sources of known pluripotent stem cells involved in the following Examples are as follows:
1. Mouse naive embryonic stem cells (mESCs):
   Purchased from Millipore Corporation.
2. Epiblast stem cells (EpiSCs):
   Prepared according to literature (Brons IG, Smithers LE, Trotter MW et al. Derivation of pluripotent epiblast stem cells from mammalian embryos. Nature 2007;448:191-195.).
3. Epiblast-like cells (EpiLCs, a kind of transiently differentiated cells):
   Prepared according to literature (Hayashi K, Ohta H, Kurimoto K, Aramaki S, Saitou M. Reconstitution of the mouse germ cell specification pathway in culture by pluripotent stem cells).
4. Rosette-like stem cells (RSCs):
   Data were obtained according to literature (Neagu A, van Genderen E, Escudero I et al. In vitro capture and characterization of embryonic rosette-stage pluripotency between naive and primed states. Nature Cell Biology 2020.).

### Example 1: Establishment of formative pluripotent stem cells (fPSCs) and human formative pluripotent stem cells (hfPSCs)

### 1. Transformation from mESCs to fPSCs or from hESCs to hfPSCs:

Preparation of fPSCs (hfPSC) medium: basal medium (KnockOut DMEM, KnockOut DMEM/F12, DMEM, DMEM/F12, neurobasal medium, or any combination thereof) supplemented with N-2 and B-27, KSR, Activin A, bFGF, a Wnt/β-catenin signal transduction inhibitor.
1) mESCs (hESCs) cell suspension was obtained;
2) mESCs (hESCs) and extracellular matrix (such as collagen) were mixed evenly and inoculated into petri dish;
3) fPSCs (hfPSCs) medium was added into the petri dish and cultured in an incubator at 37 °C to achieve the conversion of mESCs (hESCs) to fPSCs (hfPSCs).

### 2. Establish fPSCs from epiblast cells before gastrulation in vivo:

1) According to the known EpiSCs establishment method (Brons IG, Smithers LE, Trotter MW et al. Derivation of pluripotent epiblast stem cells from mammalian embryos. Nature 2007;448:191-195.) E5.5-6.5 epiblast cells were isolated from mouse embryos before and after gastrulation.
2) Epiblast cells were resuspended in fPSCs medium, mixed with extracellular matrix (such as collagen), and inoculated in petri dishes.
3) After 3-5 days, the fPSCs could be digested and passaged after forming clones.

### 3. Passage of fPSCs (hfPSCs):

Medium for passage of fPSCs (hfPSC): basal medium (KnockOut DMEM, KnockOut DMEM/F12, DMEM, DMEM/F12, neurobasal medium, or any combination thereof) supplemented with N-2 and B-27, KSR, Activin A, bFGF, a Wnt/β-catenin signal transduction inhibitor.
(1) Before passage of fPSCs (hfPSC), the petri dishes were coated with extracellular matrix;
(2) The cells and extracellular matrix were transfered to a centrifuge tube;
(3) fPSCs (hfPSCs) were collected after centrifugation;
(4) The supernatant was sucked using a vacuum pump;
(5) The fPSCs (hfPSCs) were digested into single cells;
(6) After digestion, the fPSCs (hfPSCs) were collected by centrifugation.
(8) The fPSCs (hfPSCs) were mixed with extracellular matrix (ECM) and inoculated into coated petri dishes.
(9) The fPSCs (hfPSCs) medium was added into the petri dishes and cultured in an incubator at 37 °C for passage.

### 3) Establish fPSCs from the inner cell mass

(1) ICM was extracted from mouse E3.5 blastocysts by immunosurgery.
(2) Refer to the process of establishing fPSCs from epiblast cells.

E5.5-6.5 epiblast cells were isolated from mouse embryos.
2) Epiblast cells were resuspended with fPSCs medium, mixed with extracellular matrix (such as collagen), and inoculated in petri dishes.
3) After 3-5 days, the fPSCs could be digested and passaged after forming clones.

### 3. Passage of fPSCs:

Medium for passage of fPSCs: basal medium (KnockOut DMEM, KnockOut DMEM/F12, DMEM, DMEM/F12, neurobasal medium, or any combination thereof) supplemented with N-2 and B-27, KSR, Activin A, bFGF, a Wnt/β-catenin signal transduction inhibitor.
(1) Before passage of fPSCs, the petri dishes were coated with extracellular matrix;
(2) The cells and extracellular matrix were transfered to a centrifuge tube;
(3) The fPSCs were collected after centrifugation;
(4) The supernatant was sucked using a vacuum pump;
(5) The fPSCs were digested into single cells;
(6) After digestion, the fPSCs were collected by centrifugation.
(7) The fPSCs were mixed with extracellular matrix (ECM) and inoculated into coated petri dishes.
(8) The fPSCs medium were added into the petri dishes and cultured in an incubator at 37 °C for passage.

### Example 2: Identification of basic characteristics of fPSCs

In this example, the basic characteristics of the fPSCs prepared in Example 1 were identified.

The morphology of fPSCs, mouse naive embryonic stem cells (mESCs) and epiblast stem cells (EpiSCs) prepared in Example 1 is shown in Figure 1A, wherein fPSCs could form spherical, rosette clones with smooth margins, clearly distinguishing them from the smaller domed clones of mESCs and the larger flat clones of EpiSCs.

The results of immunofluorescence staining of pluripotent stem cell markers OCT4, SOX2 and NANOG are shown in Figure 1B, the immunoblot results of the expression of various marker molecules in mESCs (under the two conditions of 2i/Lif and serum /Lif.), epiblast-like cells (EpiLCs, a kind of transiently differentiated cells), fPSCs and EpiLCs are shown in Figure 1C, wherein, unlike mESCs and EpiSCs, fPSCs specifically highly expressed the putative activation state marker Otx2. Quantitative RT-PCR results of mRNA expression levels of conjectured formative markers in mESCs, fPSCs and EpiSCs are shown in Figure 1D, fPSCs also highly expressed possible formative markers DNMT3B, SOX4 and FGF5. In addition, Figure 1E also shows the immunofluorescence results of co-staining of the conjectured formative OTX2 and cadherin in fPSCs. The above results show that the fPSCs prepared in Example 1 possess the pluripotency and immortality.

The epigenetic modification state of fPSCs was further analyzed. To detect X chromosome state, female fPSCs were established from female mESCs and stained for H3K27me3 (Plath, K., J. Fang, S.K. Mlynarczyk-Evans, R. Cao, K.A. Worringer, H. Wang, C.C. de la Cruz, A.P. Otte, B. Panning, and Y. Zhang. 2003. 'Role of histone H3 lysine 27 methylation in X inactivation', Science, 300:131-5.), the results are shown in Figure 1F, H3K27me3 immunofluorescence results show that one of the two chromosomes in female fPSCs cells is in inactivated state. The utilization of Oct4 enhancers by fPSC was evaluated through the *Oct4-ΔPE-GEP* reporting system (Illich DJ, Zhang M, Ursu A et al. Distinct Signaling Requirements for the Establishment of ESC Pluripotency in Late-Stage EpiSCs. Cell Rep2016; 15: 787-800.) and the results are shown in Figure 1G, the Oct4-GFP promoter-marked fPSCs cell line showed that, unlike OCT4utilizes a distal enhancer in mESCs, OCT4 in fPSCs utilizes a proximal enhancer.

The morphology of the hfPSC prepared in Example 1 is shown in Figures 1H and 1G. The hfPSC could form spherical and rosette clones with smooth margins. The results of immunofluorescent staining of pluripotent factors OCT4, SOX2 and NANOG and polar molecule EZRIN in human formative pluripotent stem cells (hfPSCs) are shown in Figure 1H. The immunofluorescent staining results of the expression of the formative marker Otx2 in hfPSCs are shown in Figure 1I.

### Example 3: Characteristic analysis of fPSCs at genome level

1) Sequencing: After the total RNA was extracted using the Trizol kit, it was treated with DNase I to remove residual genomic DNA. The NanoPhotometer spectrophotometer instrument, and the RNA analysis kit (Qubit RNA Assay Kit) of Qubit^{®} 2.0 Fluorometer and the RNA analysis kit (RNA 6000 Nano Kit) of the Bioanalyzer 2100 biochip analysis system were used to analyze the purity, concentration and integrity of the extracted RNA. After the quality of the sample is qualified, an average of 2 g of total RNA was prepared per sample for cDNA library construction using the NEBNext^{®} Ultra^{™} RNA Library Prep Kit. Then, Illumina Hi-Seq 2500 was used for sequencing analysis, and each sample generated 10G of raw data.
2) RNA-seq data processing: first, the reads obtained by sequencing were mapped to the mouse mm9 genome through TopHat v2.0.11 (Trapnell et al., 2009). Only reads that could not be mapped to the genome were removed. Then, based on the genome annotation information provided by refFlat, cufflinks v2.0.2 (Trapnell et al., 2009) was used to calculate the gene expression level (Fragments per kilobase per million of sequenced reads, FPKM). Data visualization was achieved by using online software provided by UCSC University (http://genome.ucsc.edu/).

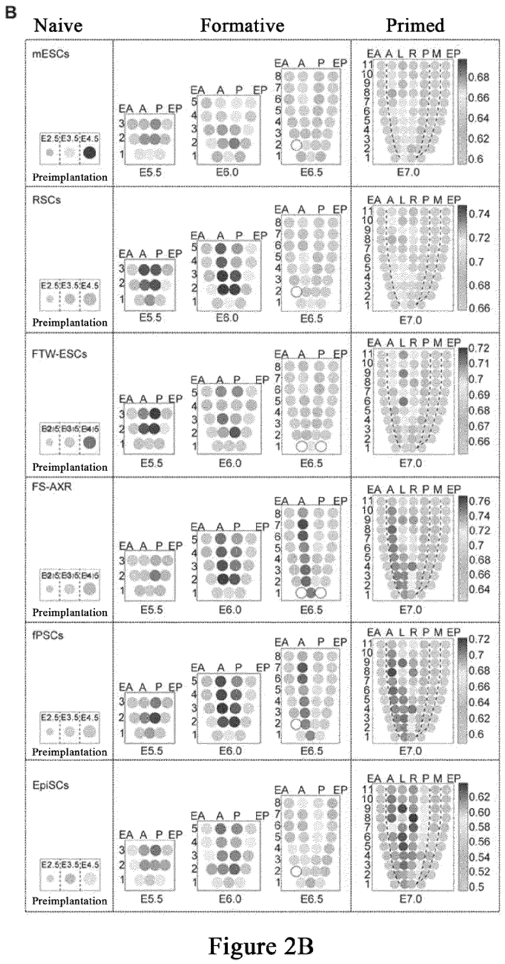

In order to detect the gene expression of the whole genome, RNA sequencing was performed on different passages of fPSCs (P1, P10, P20, P30). The results of PCA analysis are shown in Figure 2A. The results showed that genome-wide expression of fPSCs was similar to that of E6.25-E6.5 epiblast cells in vivo, but significantly different from mESCs and EpiSCs. The results of genome-wide spatiotemporal transcriptome analysis are shown in Figure 2B, and the results showed that fPSCs were most similar to E6.0 epiblast cells and part of E6.5 epiblast cells.

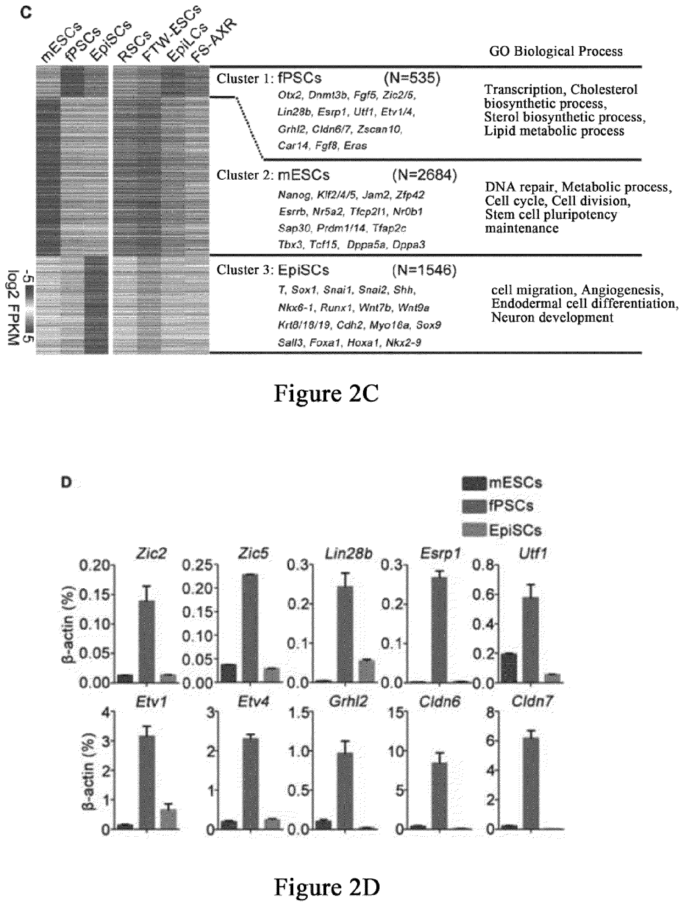

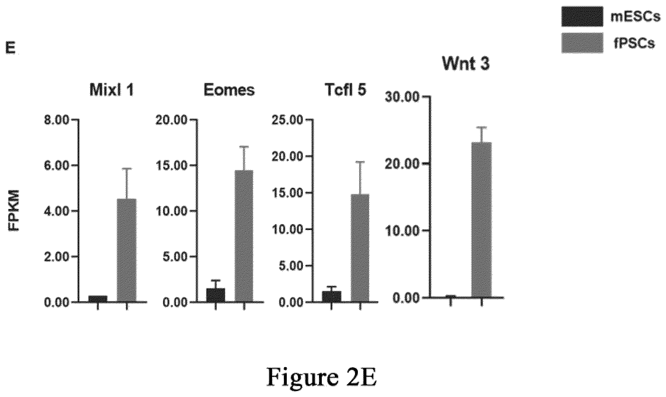

In order to obtain specific genes that can distinguish fPSCs from mESCs and EpiSCs, these cells were analyzed for differential gene expression. The results are shown in Figure 2C, fPSCs specifically highly expressed 535 genes, including previously predicted formative genes (e.g., DNMT3B, OTX2, FGF5) and many genes that have important functions in ESCs leaving the naive state, e.g., transcription factors: ZIC2/5, ETV1/4, GRH1/2, pluripotent regulators: LIN28B, UTF1, and epithelial regulators: ESRP1, CLDN6/7, etc. These genes may be marker molecules for fPSCs. The possible marker molecules of the above fPSCs were subsequently verified by quantitative RT-PCR, and the results were shown in Figure 2D and Figure 2E. Figure 2F showed transcriptome-wide association analysis of the differentiation of mESCs, EpiLCs, EpiSCs, PIfPSCs (first generation fPSCs), P10 fPSCs, P20 fPSCs, P30 fPSCs, P10 from E5.5 epiblast cells to fPSCs, P10 from E6.5 epiblast cells to fPSCs and E6. 5 epiblast cells.

### Example 4: Characteristic analysis of fPSCs at the epigenetic level

### 4.1 Analysis of epigenetic modifications (H3K4me3 and H3K27me3)

### 1) STAR ChIP-seq library construction and sequencing of H3K4me3 and H3K27me3

(1) the embryo and cell samples were transferred to 0.2 mL RNase- and DNase-free Ep tubes, and the droplet size should not exceed 0.5 µL.
(2) 48 µL of embryo lysate was taken, 2 µL of protease inhibitor (50×, Roche, Cat. No. 04693116001) was added and mixed well. 20 µl of mixed lysate was added to each reaction system, after gently pipetting using a pipette until sample lysed, and placed on ice.
(3) MNase was diluted to 0.01U with MNase working solution, 2 µL prepared MNase and 9 µL 2×MNase working solution was added to the previous step reaction, and incubated at 37°C for 5 minutes. Note that the reaction time should not be too long or too short, so as to avoid excessive digestion of chromatin by MNase or incomplete digestion reaction.
(4) 5 µL of 10×MNase stop buffer was added, pipetted gently and placed on ice.
(5) 2 µL of 10× protease inhibitor and 45 µL of pre-cooled 2×RIPA modified buffer on ice were added to the reaction in the previous step. Centrifuge at 13000 rpm at 4°C for 10 minutes.
(6) The supernatant was transferred to a new 0.2mL Ep tube, avoiding aspiration of pellets or cell debris. Then 50 µL of RIPA buffer was added.
(7) 1 µg primary antibody (self-made H3K4me3 antibody (Zhang B, Zheng H, Huang B et al. Allelic reprogramming of the histone modification H3K4me3 in early mammalian development. Nature 2016;537:553-557.); H3K27me3 antibody from Diagenode, Cat. No. C15410069) was added, and mixed by inverting overnight at 4°C.
(8) The next day, Protein A magnetic beads (Invitrogen, Cat. No. 10001D) were washed with RIPA buffer, and 100 µg of Protein A magnetic beads were added to each reaction and continued to mix by inverting at 4°C for 3 hours.
(9) The supernatant was removed on a magnet, and washed 5 times with 150 µL RIPA buffer, 5 minutes each time, afterwards, washed once with 150 µL lithium chloride washing buffer and the supernatant was discarded.
(10) 27 µL of ddH2O, 1 µL of 10×ExTaq buffer and 1 µL of proteinase K (Roche, Cat. No. 10910000) were added, and digested with shaking at 55°C for 90 minutes. After absorbing the supernatant on the magnet, it was treated at 80°C for 40 minutes to inactivate proteinase K.
(11) the reaction product of the previous step was taken out, and 1 µL of shrimp alkaline phosphatase (rSAP) was add to repair the DNA ends, and was inactivated at 85°C for 20 minutes after a hot bath at 37°C for 1 hour.
(12) 0.5 µL dCTP (1 mM) was added, and react at 95°C for 3 minutes to denature DNA, and placed on ice. Then 1 µL of terminal transferase (TDT) was added, mixed with shaking, centrifuged, and the mixture was reacted at 37°C in a PCR instrument for 35 minutes to add the nucleotide C. Then 0.5 µL dATP(1 mM) was added and incubated at 37°C for a further 5 minutes. After completion of the reaction, the TDT was inactivated at 80°C for 20 minutes.
(13) the extension reaction system was prepared according to the table below.

**Table 1-1: Extension reaction system**

| Reagent | Volume (µL) |
|---|---|
| 5×KAPA 2G buffer A | 12 |
| dNTP(2.5 mM) | 4.8 |
| Biotin MP24-G9H(2 µM) | 2 |
| KAPA 2G polymerase | 0.8 |
| H₂O | 10.2 |
| Total volume | 29.8 |

(14) 29.8 µL of extension reaction system mixture was added to the product of the previous step and PCR amplification was performed as shown in the table below.

**Table 1-2: Extension reaction program**

| Cycle | Temperature(°C) | Time(min) |
|---|---|---|
| 1 | 95 | 3 |
| 2-17 | 47 | 1 |
| | 68 | 2 |
| 18 | 72 | 10 |
| 19 | 4 | Hold |

(15) After the amplification, 6 µL of exonuclease I (Exo I) buffer and 2 µL of Exo I were added, reacted at 37°C for 50 minutes, and the residual extension primers were digested. Then Exo I was inactivated at 80°C.
(16) 22 µL 4×BW buffer was added to the product of the previous step, added 10 µL of BW-resuspended Streptavidin C1 magnetic beads (Invitrogen, Cat. No. 65001), took 5 µL from each IP reaction, washed 3 times with 1×BW after sucking the residual liquid on magnet, and re-suspended to 10 µL). It was then transferred to a 1.5 mL Ep tube and shaken at 1100 rpm for 25 minutes at 23 °C to bind DNA to C1 megnetic beads.
(17) After removing the waste liquid on the magnet, 150 µL of 1×BW buffer was added to wash once, and then washed three times with EBT buffer. Then the DNA was eluted by using 8.8µL EB buffer.
(18) The ligation reaction system was prepared as shown in the table below, 11.2 µL of the ligation reaction system mixture was added to the eluted DNA, and inverted overnight at 4°C.

**Table 1-3: Ligation reaction system**

| Reagent | Volume(µL) |
|---|---|
| 2×quick ligation buffer(NEB) | 10 |
| TA adapter(10 mM) | 0.2 |
| Quick ligase | 1 |
| Total volume | 11.2 |

(19) The next day, the overnight ligation product was take out, after 10 minutes at room temperature, the liquid was removed on a magnet, washed once with 1×BW and three times with EBT, then 30 µL H2O was added, shaken at 72°C 1400 rpm for 30 minutes, and then the DNA product was eluted on a magnet.
(20) the amplification reaction system was prepared according to the table below.

**Table 1-4: Amplification reaction system**

| Reagent | Volume(µL) |
|---|---|
| 10×Ex Taq buffer | 5 |
| dNTP(2.5 mM) | 5 |
| P1_FL(20 µM) | 0.7 |
| Ex Taq HS polymerase | 0.5 |
| H2O | 8.1 |
| Index(20 µM) | 0.7 |
| Total volume | 20 |

(21) 20 µL of the amplification system mixture was added to the DNA product of the previous step, and the PCR amplification reaction was carried out as shown in the table below.

**Table 1-5: Amplification PCR program**

| Cycle | Temperature(°C) | Time(min) |
|---|---|---|
| 1 | 95 | 3 |
| 2-17 | 95 | 30s |
| | 58 | 30s |
| | 70 | 1 |
| 18 | 72 | 7 |
| 19 | 4 | Hold |

(22) Ampure magnetic beads (Beckman, product number A63882) were used to purify DNA at a ratio of 1:1, and the concentration was measured using Qbit after dissolving in 40 µL H₂O. Then sequencing was carried out on HiSeq2500 or XTen.

### 2. STAR ChIP-seq data processing and identification of super bivalent genes

Data processing: First, the reads (Reads) obtained by sequencing were mapped to the mouse mm9 genome by Bowtie2v2.2.2 (Langmead B, Salzberg SL. Fast gapped-read alignment with Bowtie 2. Nat Methods 2012;9:357-359.), the parameters were set to -t -q -N 1 -L 25. Second, reads that cannot be mapped to the genome, reads that can map to multiple positions, and duplicated reads caused by PCR were removed. After verifying the reproducibility between biological replicates, the data from different replicates were integrated. Afterwards, the whole genome was divided into 100bp length sequence frames, the signal enrichment in each 100bp region was counted, and the signal was normalized using RPKM (Reads per kilobase per million of sequenced reads). Data visualization was achieved by using online software provided by UCSC University (http://genome.ucsc.edu/).

Identification of super bivalent genes: refer to our previously published papers (Xiang Y, Zhang Y, Xu Q et al. Epigenomic analysis of gastrulation identifies a unique chromatin state for primed pluripotency. Nat Genet 2020;52:95-105.). Briefly, firstly, based on the published distribution of H3K4me3 and H3K27me3 in 27 different tissue samples, combined with gene expression, 3992 bivalent genes (co-labeled by H3K4me3 and H3K27me3 in at least half of the samples, and gene expression FPKM value < 2) and 3181 housekeeping genes (expressed in all samples, FPKM > 2) were screened out; secondly, the enrichment degree of H3K4me3 and H3K27me3 signals within the 10-kb range of the promoter region in each sample was calculated and normalized by using the Z-score method; finally, among all bivalent genes, super bivalent genes were screened according to H3K4me3 and H3K27me3 signals, and the screening criteria were: 1) the enrichment signal of H3K4me3 in the promoter region was stronger than that of housekeeping gene; 2) the promoter region has a strong H3K27me3 modification (RPKM>1 after normalization).

### 3. DNA methylation sequencing and analysis

10⁷ cells were collected and sent to Novogene after quick frozen in liquid nitrogen, then performed DNA methylation library construction and genome-wide disulfide bond sequencing (30G data volume) according to the company's standard process. Using the BSseeker2 software developed by Michael Zhang's lab (Guo et al., 2013), reads were mapped to the mouse mm9 genome with the parameters --bt2-p 8 --XS 0.2,3-a CCCCCC -m 4. Reads that could not be mapped to the genome, reads that could map to multiple locations, and duplicated reads caused by PCR removed. Afterwards, the methylation ratio of a single base C on the genome was calculated separately, and only the C sites that were detected to be more than 3 on the genome were retained, and the average DNA methylation degree per 100 bp of the whole genome is calculated.

### 4. Results:

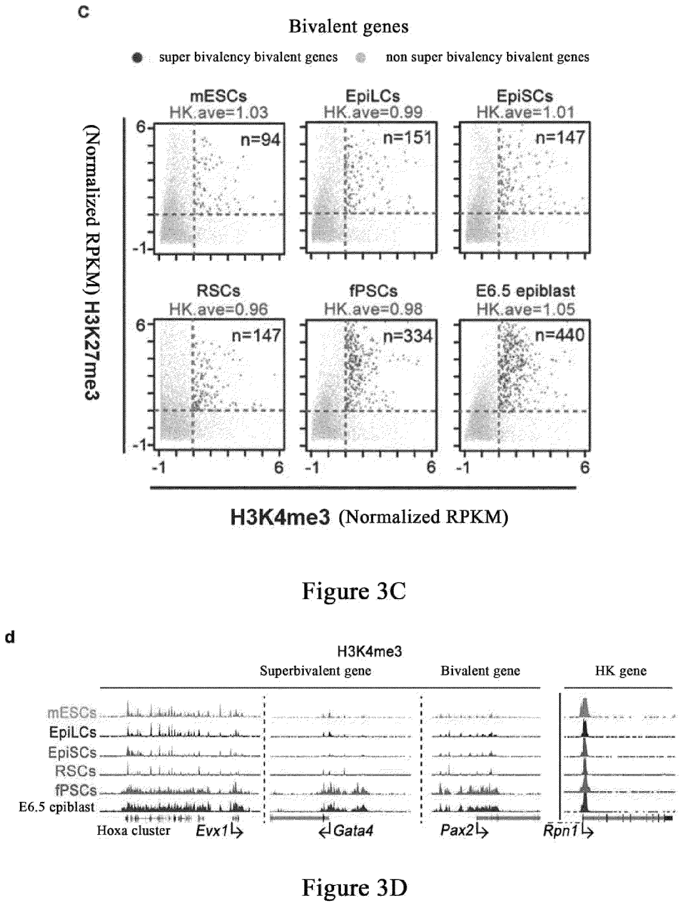

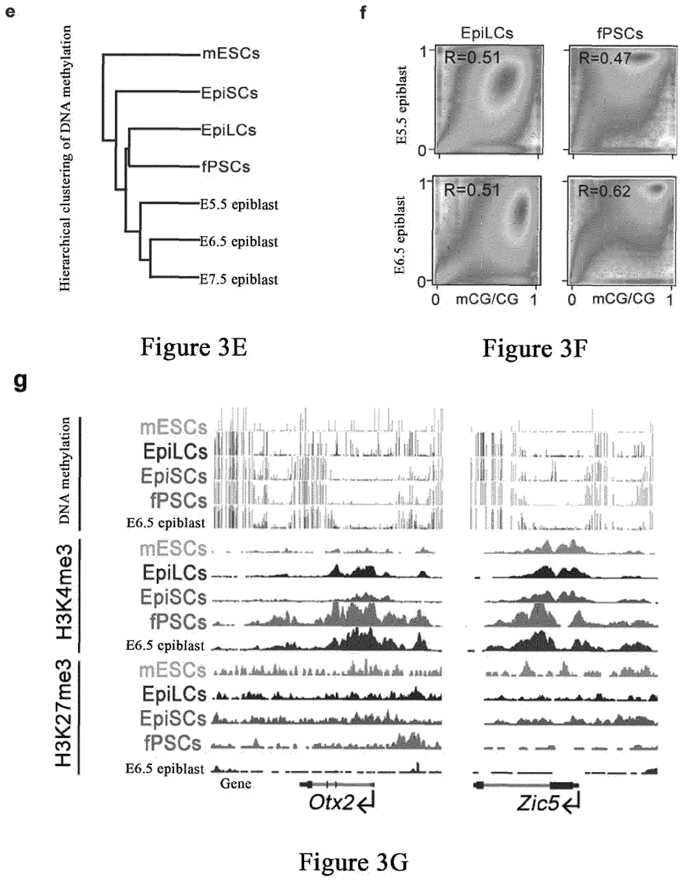

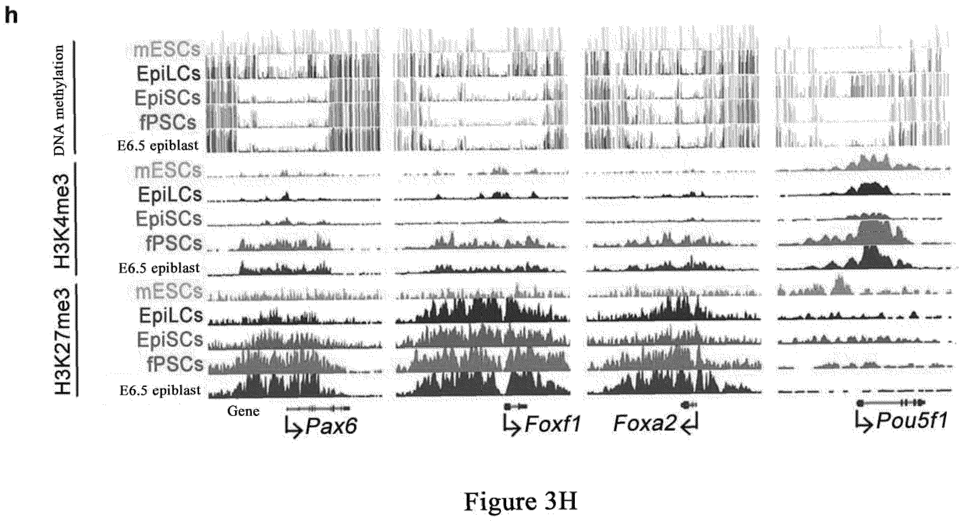

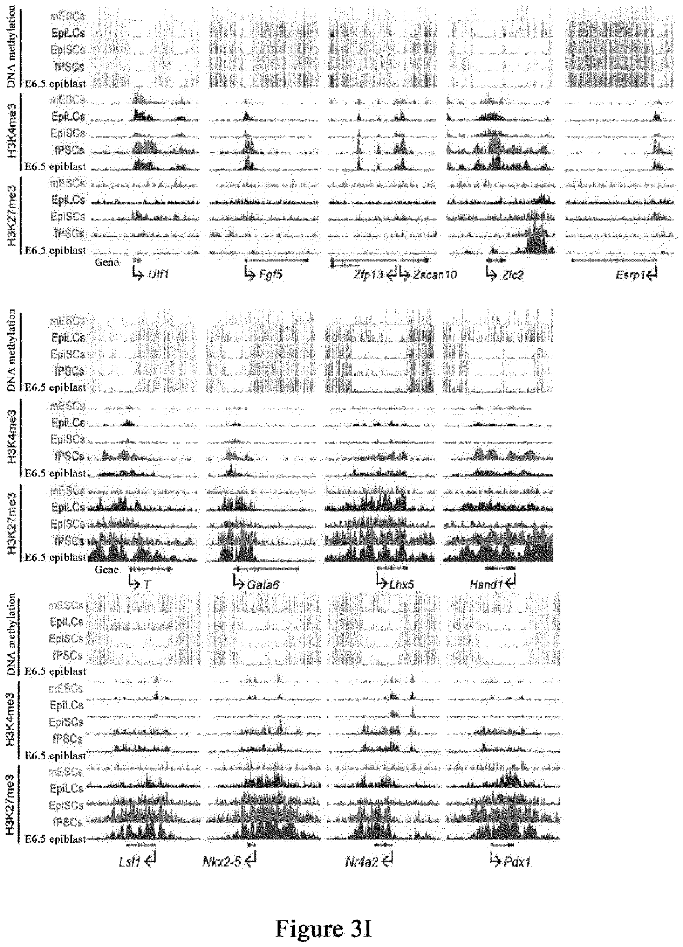

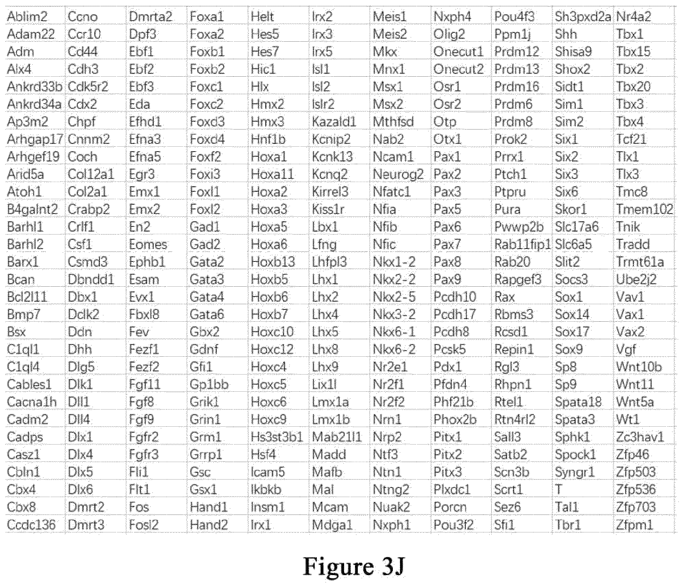

The results of epigenetic modification (H3K4me3 and H3K27me3) and DNA methylation sequencing and analysis are shown in Figure 3. Figure 3A shows the distribution of H3K4me3 and H3K27me3 epigenetic modifications on promoters (-2.5Kb) at genome-wide level in various stem cells. Wherein, for fPSC, RSC, and E6.5 epiblast cells, the distribution of H3K27me3 in the promoter region of all genes is stronger than that of other pluripotent stem cells, but the distribution of H3K4me3 in the promoter region of all genes does not show obvious difference. Figure 3B shows the distribution of H3K4me3 of the promoters (upstream or downstream 20Kb) at the genome-wide level among super bivalent genes, housekeeping genes and bivalent genes. The results show that fPSCs were most similar to the epiblast cells isolated in vivo in terms of super bivalent genes. Figure 3C shows the number of super bivalent genes identified in different pluripotent stem cells, and the results show that the number of super bivalent genes in fPSCs (334) was more than twice that of other in vitro stem cell lines (94-151), and was closest to in vivo E6.5 epiblast cells (440). 94% of the super bivalent genes identified in fPSCs were also identified in E6.5 epiblast cells, for example, super bivalency of Hoxa cluster, Evx1, Gata2, Pax6 were only found in fPSCs and E6.5 epiblast cells, but not in other pluripotent stem cells (Figure 3D). Figures 3E-F show the detection results of methylation level at the genome-wide level, and the results showed that fPSCs were closest to in vivo E6.5 epiblast cells compared with other cells.

The epigenetic modification (H3K4me3 and H3K27me3) and DNA methylation of specific genes were further analyzed. Figures 3G-3I show the distribution of epigenetic modifications of formative genes and cell lineage marker molecules of the three germ layers. Figure 3G shows DNA methylation and H3K4me3/H3K27me3 enrichment at promoters of formative maker (Otx2, Zic5). Figure 3H shows DNA methylation and enrichment of H3K4me3 and H3K27me3 at the promoters of different germ layer makers (Pax6, Foxf1 and Foxa2) and Oct4. Figure 3I shows DNA methylation and H3K4me3/H3K27me3 enrichment at promoters of Utf1, Fgf5, Zfp13, Zscan10, Zic2, Esrp1, T, Gata6, Lhx5, Hand1, Isl1, Nkx2-5, Nr4a2, Pdx1. Figure 3J shows the list of super bivalent genes of fPSCs.

The results show that in fPSCs, the promoter regions of formative genes (Otx2, Zic5, Utf1, Fgf5, Zfp13, Zscan10, Zic2, Esrp1, etc.) all had higher levels of transcriptional activation markers H3K4me3 and lower levels of transcriptional repression markers H3K27me3, and low levels of DNA methylation, similar to E6.5 day epiblast cells; the promoter region of lineage-specific genes (Hand1, T, Foxa2, Nkx2-5, Pax6, PDX1, Isl1, Tcf21, Lhx5, Pax2, Dlx5, Nr4a2, etc.) has high levels of H3K4me3 and H3K27me3, and low levels of DNA methylation.

The above results showed that, compared with other pluripotent stem cells (including RSCs), the fPSC of the present application is closest to E6.5 day epiblast cells at the epigenetic level.

### Example 5: Differentiation of fPSCs

1. Differentiation of fPSCs to primordial germ cell-like cells (PGCLCs):
   Preparation of PGCLCs medium: GMEM basal medium, 15% KSR, 1mM pyruvic acid, 0.1mM non-essential amino acids, 0.1mM β-mercaptoethanol, 2mM L-glutamine, 100 U/ml penicillin, 0.1mg/ml streptomycin, BMP4: 500ng/ml, SCF: 100ng/ml, BMP8a: 500ng/ml, EGF: 50ng/ml, and hLIF: 1000u/ml;
   1) The clones of fPSCs were collected and digested into single cells by TrypLE;
   2) The cells were washed twice with 15% KSR medium, and after cell counting, the cells were resuspended and inoculated in a 96-well plate with PGCLCs medium at a density of 2000 cells/96-well plate.
2. Differentiation of fPSCs to ectoderm:
   1) When the continuously passaged fPSCs were cultured to the third day, the medium was replaced with N2B27 medium, and 6 µM PD0325901 was added thereto;
   2) After 24 hours, PD0325901 was removed and the medium was replaced with N2B27 basal medium. After continuous culture for 24 hours, the neural precursor cells of Sox1-GFP could be obtained, and then replaced with fresh N2B27 medium every day;
   3) Neural stem cells (Nestin⁺) could be obtained on the fourth day after differentiation, and terminally differentiated neurons (Tuj1⁺) could be obtained on the seventh day.
3. Differentiation of fPSCs to mesoderm:
   BM1 medium: RPMI 1640 medium; 4mM Vitamin C, B27 supplement minus insulin; BM2 medium: 10µM XAV939 was add on the basis of BM1;
   1) When the continuously passaged fPSCs were cultured to the third day, the medium was replaced with EpiLCs medium, and cultured for 24 hours, so that the fPSCs underwent EMT, and the clones changed from a regular spherical shape to an irregular shape;
   2) On the second day after induction of cardiomyocyte differentiation, the medium was replaced with BM1 medium;
   3) On the third day of differentiation, replaced with BM2 medium;
   4) After 24 hours, BM2 was replaced with BM1, and the clones began to beat one after another after replacement with BM1. On the fifth day of differentiation, more than 90% of the clones have started to beat.
4. Differentiation of fPSCs to endoderm:
   1) When the continuously passaged fPSCs were cultured to the third day, the medium was replaced with the EpiLCs medium, and cultured for two days;
   2) After 48 hours, the medium was replaced with RPMI 1640 medium, B27supplement minus insulin and 100ng/ml Activin A were added to it to continue culturing for 2 days. At this time, a high proportion of Foxa2-positive primitive endoderm cells could be obtained;
   3) Then, the medium was changed to fresh RPMI 1640 medium, and B27 supplement, 10 ng/ml bFGF and 20 ng/ml BMP4 were added to it, the cells were transferred to a low-oxygen (5% CO₂/4% O₂) environment and cultured for 2 days, replacing with fresh medium every day;
   4) For the next 5 days, the medium was replaced with fresh RPMI 1640 medium, to which 2.5% fetal bovine serum (ES-009-B) and B27 supplement, as well as 100 nM Dexamethasone, 10 ng/ml HGF, and 20 ng/ml Oncostatin M were added, and the culture was continued in a hypoxic environment. Thus, liver precursor cells with positive AFP antibody staining were obtained.
5. Results

To investigate the ability of fPSCs to differentiate into PGCLCs, BVSC fPSCs were constructed from BVSC mESCs according to literature (Hayashi K, Ohta H, Kurimoto K, Aramaki S, Saitou M. Reconstitution of the mouse germ cell specification pathway in culture by pluripotent stem cells. Cell 2011;146:519-532., Ohinata Y, Payer B, O'Carroll D et al. Blimp1 is a critical determinant of the germ cell lineage in mice. Nature 2005;436:207-213.), all of which were negative for BVSCs, subsequently, BVSC fPSCs were induced to differentiate into PGCLCs by the method described in 1 above, and unstable epiblast-like cells (EpiLCs) were used as control. Differentiation results are shown in Figures 4A-B. The results in Figure 4A show that fPSCs at the 20^{th} passage were still able to differentiate into BV+SC+ cells, indicating that long-term proliferation hardly affected the ability of fPSCs to differentiate into PGCLCs. The results in Figure 4B show that the differentiation efficiency of fPSCs into PGCLCs (SSEA1 and CD61 double-positive cells) with potential function is 11%, which is close to the unstable epiblast-like cells (EpiLCs) with the best differentiation efficiency known at present (12.3%).

To investigate the ability of fPSCs to differentiate into neuroectoderm, GFP-negative *Sox1-GFP* fPSCs were obtained from mESCs reported by 46C (Yu Y, Wang XX, Zhang XX et al. ERK inhibition promotes neuroectodermal precursor commitment by blocking self-renewal and primitive streak formation of the epiblast. Stem Cell Res Ther 2018; Ying QL, Stavridis M, Griffiths D, Li M, Smith A. Conversion of embryonic stem cells into neuroectodermal precursors in adherent monoculture. Nature biotechnology 2003; 21:183-186.), followed by induction of differentiation into neuroectoderm by the method described in 2 above, with *Sox1-GFP* EpiLC and ESC monolayer differentiation systems as controls, GFP-positive cells were determined by flow cytometry (Yu Y, Wang XX, Zhang XX et al. ERK inhibition promotes neuroectodermal precursor commitment by blocking self-renewal and primitive streak formation of the epiblast. Stem Cell Res Ther 2018; Ying QL, Stavridis M, Griffiths D, Li M, Smith A. Conversion of embryonic stem cells into neuroectodermal precursors in adherent monoculture. Nature biotechnology 2003;21:183-186.). The differentiation results are shown in Figures 4C-D. The results in Figure 4C show that the differentiation efficiency of fPSCs to neural precursor cells (Sox1 positive) was as high as 94% the next day, which was significantly higher than the 72.6% of EpiLCs; and compared with the ESC monolayer differentiation system (68%), not only the efficiency was high, but also the time was much shorter (5-6 days), in addition to being more synchronized. The results in Figure 4D show that the neural precursor cells derived from fPSCs could form neuron-like cells after 2 more days of differentiation.

To investigate the ability of fPSCs to differentiate into endoderm, the differentiation into hepatocyte-like cells was induced by the method described in 4 above, then endoderm marker Foxa2 and liver cell marker AFP were stained. The differentiation results are shown in Figures 4E-F. The results in Figure 4E show that the differentiation efficiency of fPSCs to endoderm precursor cells (Foxa2) reached 88%. The results in Figure 4F show that fPSCs-derived endoderm precursor cells could differentiate into hepatocyte-like cells after another 7 days.

In order to investigate the ability of fPSCs to differentiate into mesoderm, the method described in 3 above was used to induce differentiation into cardiomyocyte-like cells, and the differentiation of embryonic stem cells to EB was used as control. The results are shown in Figures 4G-J. The results in Figure 4G show that the differentiation efficiency of fPSCs differentiating into mesoderm cells (beating cardiomyocyte-like cells) reached 90%, significantly higher than the differentiation efficiency of ESCs into cardiomyocyte-like cells through conventional EB differentiation (~60%). The results in Figure 4H show that cardiomyocyte-like cells derived from fPSCs expressed the proliferation marker molecule Ki67, and also expressed cardiomyocyte marker molecules (MLC, α-actinin, cTnT, and Gata4); in addition, sarcomeres were clearly visible. The results in Figures 4I-J show that after 3-4 days of maturation of fPSCs-derived cardiomyocyte-like cells, calcium ion release (I) and electrophysiological activity (J) could be detected.

The above results indicate that fPSCs of the present application have a greatly improved ability to efficiently differentiate into all cell lineages of the embryo compared to embryonic stem cells. Without being bound by theory, it is believed that ESCs are in a state that is not ready to differentiate, and similar to pre-implantation epiblast cells, these cells need to go through a maturation process in the genomic, epigenomic, metabolic and other regulatory pathways to achieve the "high potential energy" cell state (formative state) immediately before the initiation of gastrulation, and the fPSCs of the present application may correspond to this state. Therefore, fPSCs may be the only way to efficiently differentiate ESCs and iPSCs into functional cells in the future, and they have important application value in directed differentiation of stem cells and organoids.

### Example 6: Analysis of characteristics of hfPSCs at genome level

1) Sequencing: hfPSCs were subjected to RNA-Seq by the method of Example 3
2) RNA-seq data processing: first, the reads obtained by sequencing were mapped to the human hg38 genome through Hisat2v2.2.1. Only reads that could not be mapped to the genome were removed. Then, based on the genome annotation information provided by refFlat, counts of gene expression levels were calculated using featureCount. The relative expression of sample genes was calculated by TPM (Transcripts PerKilobase Million) standardization method. Data visualization was achieved by using online software provided by UCSC University (http://genome.ucsc.edu/).

In order to detect the gene expression of the whole genome, hfPSCs were subjected to RNA sequencing, and the results of PCA analysis are shown in Figure 5A. The results show that the genome-wide expression pattern of fPSCs is between naive hESCs and primed hESCs, and the pluripotent state is activated state (formative state), which is significantly different from FS (Smith et al., 2020) and XPSC (Wu et al., 2020).

In order to obtain specific genes that can distinguish hfPSCs from naive hESCs and primed hESCs, differential gene expression analysis was performed on these cells. As shown in Figure 5B, hfPSCs specifically highly expressed 2378 genes, which may be marker molecules of hfPSCs. There are 84 formative genes in the intersection of hfPSCs and fPSCs, and these genes may be the conserved marker molecules shared by fPSCs and hfPSCs. The biological process cluster analysis of the genes highly expressed in hfPSCs is shown in Figure 5C. The high level of cholesterol, sterol metabolism and fat metabolism of hfPSCs are unique to the formative state, which is consistent with the metabolic characteristics of mouse fPSCs; suggesting that this metabolic level of formative pluripotency may be conserved in humans and mice.

### Example 7: Analysis of characterization of hfPSCs at the epigenetic level

### 7.1 Analysis of epigenetic modifications (H3K4me3 and H3K27me3)

1. The STAR ChIP-seq library construction and sequencing of H3K4me3 and H3K27me3 were carried out on hfPSC by the method of Example 4.
2. STAR ChIP-seq data processing and identification of super bivalent genes
   Data processing: first, the reads obtained by sequencing were mapped to the human hg38 genome by Bowtie2 v2.2.2 (Langmead B, Salzberg SL. Fast gapped-read alignment with Bowtie 2. Nat Methods 2012;9:357-359.). Second, reads that could not be mapped on the genome, reads that could map to multiple positions, and duplicate reads caused by PCR were removed. After verifying the reproducibility between biological replicates, the data from different replicates were integrated. Afterwards, the whole genome was divided into 100bp length sequence frames, and the signal enrichment in each 100bp region was counted, and the signal was normalized using RPKM (Reads per kilobase per million of sequenced reads). The data visualization was achieved by using online software (https://epigenomegateway.wustl.edu/browser/) provided by wash u. The identification of super bivalent genes was carried out by the method of Example 4.
3. Results:
   Figure 6A shows the number of super bivalent genes identified in hfPSCs. The results show that the number of super bivalent genes in hfPSCs is 174, and there are 31 super bivalent modification genes in the intersection of hfPSCs and fPSCs. These genes may be the super bivalent modification genes shared by fPSCs and hfPSC. Figure 6B shows the super covalent modification patterns of H3K4me3 and H3K27me3 at promoter regions of HOXA cluster, FOXA2, PAX6, FOXF2, GDNF, IRX1, MSX1, NFIB, NKX3-2, NRN1, PAX5, PTGER4, PITX3, SLIT2, TBX1, VLDLR, EVX1, PHOX2B genes in hfPSCs.

Although the specific implementation of the present application has been described in detail, those skilled in the art will understand that: according to all the data and methods that have been published, various modifications and changes can be made to the details, and these changes are all within the protection scope of the present application. The full scope of the present application is given by the claims appended hereto and any equivalents thereof.

## Claims

1. An isolated pluripotent stem cell, having the following characteristics:
(i) having the pluripotency of a mammalian embryonic epiblast cell from post-implantation to about to undergo gastrulation (early post-implantation epiblast);
(ii) having the formative differentiation function, and optional morphological characteristic and/or genomic and/or epigenomics characteristic;
(iii) being capable of stable passage for at least 5 times, such as at least 10 times, at least 15 times, at least 20 times, at least 25 times, at least 30 times or more.

2. The pluripotent stem cell of claim 1, which is capable of differentiating into a germ cell or a germ precursor cell.

3. The pluripotent stem cell of claim 1 or 2, which is capable of differentiating into an endoderm, ectoderm or mesoderm cell under a condition that allows the differentiation of the pluripotent stem cell, and the differentiation efficiency is at least 50%, at least 60%, at least 70% %, at least 80%, at least 90% or more.

4. The pluripotent stem cell of any one of claims 1-3, which is capable of differentiating into a germ cell or a germ precursor cell under a condition that allows the differentiation of the pluripotent stem cell, and the differentiation efficiency is at least 10% or more.

5. The pluripotent stem cell of any one of claims 1-4, which is capable of differentiating into an endoderm, ectoderm or mesoderm cell within five days, four days, three days, two days or one day after applying a differentiation condition, and the differentiation efficiency is at least 20%, at least 30%, at least 40%, at least 50%, at least 60%, at least 70%, at least 80%, at least 90% or higher.

6. The pluripotent stem cell of any one of claims 1-5, which is capable of forming an epiblast-like embryoid (EpiBlastoid) with a rosette-like structure.

7. The pluripotent stem cell of any one of claims 1-6, having one or more of the following characteristics:
(i) compared with the expression level of these genes in an embryonic stem cell, the expression level of one or more (e.g., 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71) genes selected from the following in the pluripotent stem cell shows at least about 2 times increase: CLDN6, WNT3, MIXL1, EOMES, TCFL5, ZIC5, LIN28B, GRHL2, MYCN, ABHD14A, RPUSD2, MVK, SPRED2, E2F3, TFAP4, HGH1, PPIF, TFDP1, PRSS8, SLC9A3R1, IFRD2, KDF1, ARRB2, KLHL23, TSPAN5, RAB25, CLDN6, DHCR7, RBM47, KHK, SMCO4, NABP2, RBPMS2, TMEM39B, REPIN1, MVD, SOX3, RARG, TMEM30B, IGSF9, CDH24, LSS, MARVELD3, ADGRB2, EOMES, ZCCHC3, SLC39A8, ZSCAN10, FADS2, NECTIN1, SNAI3, MOXD1, MYC, PDZD4, SLC7A8, ETV4, SEPHS1, ZIC2, JPH4, TMEM125, MPLKIP, LSM2, ZIC5, SLC27A2, MMP25, DERL3, TMEM59L, EPHA1, FOXD3, ETV5;
(ii) the pluripotent stem cell comprises at least 200, at least 250 or at least 300 super bivalent genes;
(iii) the pluripotent stem cell has super bivalency in the promoter region of one or more (e.g., 1, 2, 3, 4, 5, 6, 7, 8, 9, 10, 11 1, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, or all 43) genes selected from the following: HAND1, BRACHYURY/T, FOXA2, NKX2-5, PAX6, PDX1, ISL1, TCF21, LHX5, PAX2, DLX5, NR4A2; ABLIM2, ANKRD33B, BARX1, DMRT2, DMRT3, EVX1, FGFR3, FLT1, FOXC1, FOXF2, GDNF, HOXA11, HOXA3, HOXA5, HOXA6, IRX1, IRX2, KISS1R, MSX1, NFIB, NFIC, NKX3-2, NRN1, OTP, PAX5, PHOX2B, PITX3, PTGER4 , SLIT2, TBX1, VLDLR;
(iv) compared with a promoter region of the corresponding genes in an embryonic stem cell, the promoter region of one or more (e.g., 2, 3, 4, 5, 6, 7 or all 8) genes selected from the following in the pluripotent stem cell shows increased H3K4me3 level and/or decreased H3K27me3 level: OTX2, ZIC5, UTF1, FGF5, ZFP13, ZSCAN10, ZIC2, ESRP1.

8. The pluripotent stem cell of any one of claims 1-7, compared with the expression level of these genes in an embryonic stem cell (ESC), the expression level of one or more (e.g., 2, 3, 4, or all 5) genes selected from the following in the pluripotent stem cell shows at least about 2 times, at least about 3 times, at least about 4 times, at least about 5 times, at least about 6 times, at least about 7 times, at least about 8 times, at least about 9 times or about 10 times increase: CLDN6, WNT3, MIXL1, SOX4, LIN28B, EPHA1, JPH4, MMP25, MOXD1, MYC, NECTIN1, SLC39A8, SLC7A8, TMEM125, TMEM59L, ZIC2, ZIC5.

9. The pluripotent stem cell of any one of claims 1-8, having one or more of the following characteristics:
(1) the expression level of EOMES in the pluripotent stem cell is at least about 2 times, at least about 3 times, at least about 4 times or about 5 times that of the gene in an embryonic stem cell;
(2) the expression level of TCFL5 in the pluripotent stem cell is at least about 2 times, at least about 3 times, at least about 4 times, at least about 5 times, at least about 6 times, at least about 7 times, at least about 8 times, at least about 9 times or about 10 times that of the gene in an embryonic stem cell;
(3) the expression level of ZIC5 in the pluripotent stem cell is at least about 2 times, at least about 3 times, at least about 4 times, at least about 5 times, at least about 6 times, at least about 7 times, at least about 8 times, at least about 9 times or about 10 times that of the gene in an embryonic stem cell;
(4) the expression level of FOXH1 in the pluripotent stem cell is at least about 2 times, at least about 3 times, at least about 4 times or about 5 times that of the gene in an embryonic stem cell;
(5) the expression level of GRHL2 in the pluripotent stem cell is at least about 2 times, at least about 3 times, at least about 4 times or about 5 times that of the gene in an embryonic stem cell;
(6) the expression level of MYC, MOXD1, NECTIN1, SLC39A8, SLC7A8, ZIC2 in the pluripotent stem cell is at least about 2 times, at least about 3 times, at least about 4 times or about 5 times that of the genes in an embryonic stem cell;
(7) the expression level of EPHA1, JPH4, MMP25, TMEM125, TMEM59L in the pluripotent stem cell is at least about 2 times, at least about 3 times, at least about 4 times, at least about 5 times, at least about 6 times, at least about 7 times, at least about 8 times, at least about 9 times or about 10 times that of the genes in an embryonic stem cell.

10. The pluripotent stem cell of any one of claims 1-9, wherein the expression level is mRNA level or protein level;
preferably, the expression level is mRNA level.

11. The pluripotent stem cell of any one of claims 1-10, which has super bivalency in the promoter region of at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, or 31 genes selected from the following: ABLIM2, ANKRD33B, BARX1, DMRT2, DMRT3, EVX1, FGFR3, FLT1, FOXC1, FOXF2, GDNF, HOXA11, HOXA3, HOXA5, HOXA6, IRX1, IRX2, KISS1R, MSX1, NFIB, NFIC, NKX3-2, NRN1, OTP, PAX5, PHOX2B, PITX3, PTGER4, SLIT2, TBX1, VLDLR.

12. The pluripotent stem cell of any one of claims 1-10, which has super bivalency in the promoter region of at least 200, at least 250 or at least 300 genes selected from the following: ABLIM2, ADAM22, ADM, ALX4, ANKRD33B, ANKRD34A, AP3M2, ARHGEF26, ARID5A, ATOH1, B4GALNT2, BARHL1, BARHL2, BARX1, BCAN, BCL2L11, BMP7, BSX, C1QL1, C1QL4, CACNA1H, CADPS, CAMK2D, CASZ1, CBLN1, CBX4, CBX8, CCNO, CCR10, CD44, CDH3, CDX2, CEBPD, CHPF, CNNM2, COCH, COL12A1, COL2A1, CRLF1, CSF1, CSMD3, DBNDD1, DBX1, DCLK2, DDHD1, DDN, DHH, DLG5, DLK1, DLL1, DLL4, DLX1, DLX4, DLX5, DLX6, DMRT2, DMRT3, DMRTA2, DPF3, EBF1, EBF2, EBF3, EDA, EFHD1, EFNA3, EGR3, EMX1, EMX2, EN2, EOMES, EPHA7, EPHB1, ESAM, EVX1, FBXL8, FEV, FEZF1, FEZF2, FGF11, FGF8, FGF9, FGFR2, FGFR3, FLI1, FLT1, FOS, FOSB, FOSL2, FOXA1, FOXA2, FOXB1, FOXB2, FOXC1, FOXC2, FOXD3, FOXD4, FOXF2, FOXI3, FOXL1, FOXL2, FZD10, GAD1, GAD2, GATA2, GATA3, GATA4, GATA6, GBX2, GDNF, GFI1, GP1BB, GRIK1, GRIN1, GRK4, GRM1, GRRP1, GSC, GSX1, HAND1, HAND2, HELT, HES5, HES7, HIC1, HLX, HMX2, HMX3, HNF1B, HOXA1, HOXA11, HOXA2, HOXA3, HOXA5, HOXA6, HOXB13, HOXB5, HOXB6, HOXB7, HOXC10, HOXC12, HOXC4, HOXC5, HOXC6, HOXC9, HS3ST3B1, HSD17B1, HSF4, ICAM4, ICAM5, INSM1, IRX1, IRX2, IRX3, IRX5, ISL1, ISL2, ISLR2, JAK3, KAZALD1, KCNK13, KCNQ2, KIRREL2, KIRREL3, KISS1R, LBX1, LEFTY1, LFNG, LHFPL3, LHX1, LHX2, LHX4, LHX5, LHX8, LHX9, LIPG, LIX1L, LMX1A, LMX1B, LRBA, MAB21L1, MAB21L2, MAFB, MAL, MDGA1, MEIS1, MEIS2, MID1IP1, MKX, MNX1, MSX1, MSX2, MTHFSD, NAB2, NCAM1, NEUROG2, NFATC1, NFIA, NFIB, NFIC, NKX1-2, NKX2-2, NKX2-5, NKX3-2, NKX6-1, NKX6-2, NR2E1, NR2F1, NR2F2, NR4A2, NRN1, NRP2, NTN1, NTNG2, NUAK2, NXPH1, NXPH4, OLIG2, ONECUT1, ONECUT2, OSR1, OSR2, OTP, OTX1, PAX1, PAX2, PAX3, PAX5, PAX6, PAX7, PAX8, PAX9, PCDH10, PCDH17, PCDH7, PCDH8, PCDHGC4, PCDHGC5, PCSK5, PDX1, PHF21B, PHOX2B, PITX1, PITX2, PITX3, PLEKHH3, PLXDC1, PORCN, POU3F2, POU4F3, PPM1J, PRDM12, PRDM13, PRDM16, PRDM6, PRDM8, PROK2, PRRX1, PTGER4, PTPRU, PURA, RAPGEF3, RAX, RBM3, RBMS3, RBMX, RCSD1, REPIN1, RHPN1, RTN4RL2, SALL3, SATB2, SCN3B, SCRT1, SEZ6, SFI1, SH3PXD2A, SHH, SHISA9, SHOX2, SIDT1, SIM1, SIM2, SIX1, SIX2, SIX3, SIX6, SKOR1, SLC17A6, SLC25A25, SLC6A5, SLIT2, SOCS3, SOX1, SOX14, SOX17, SOX9, SP8, SP9, SPATA18, SPATA3, SPHK1, SPOCK1, SYNGR1, T, TAL1, TBR1, TBX1, TBX15, TBX2, TBX20, TBX3, TBX4, TCF21, TLX1, TLX3, TMEM102, TNIK, TRADD, TRIM67, TRMT61A, VAV1, VAX1, VAX2, VGF, VLDLR, WNT10B, WNT11, WNT5A, WT1, ZC3HAV1, ZFP46, ZFP503, ZFP536, ZFP703.

13. The pluripotent stem cell of any one of claims 1-12, compared with the promoter region of the corresponding genes in an embryonic stem cell, the promoter region of one or more (e.g., 2, 3, 4, 5, 6, 7, 8, 9, 10, 11 or all 12) genes selected from the following in the pluripotent stem cell shows lower DNA methylation level: HANOI, T, FOXA2, NKX2-5, PAX6, PDX1, ISL1, TCF21, LHX5, PAX2, DLX5, NR4A2.

14. The pluripotent stem cell of any one of claims 1-13, compared with the promoter region of the corresponding genes in an embryonic stem cell, the promoter region of one or more (e.g., 2, 3, 4, 5, 6, 7 or all 8) genes selected from the following in the pluripotent stem cell shows lower DNA methylation level: OTX2, ZIC5, UTF1, FGF5, ZFP13, ZSCAN10, ZIC2, ESRP1.

15. The pluripotent stem cell of any one of claims 1-14, wherein the pluripotent stem cell is derived from an embryo before and after gastrulation in mammals.

16. The pluripotent stem cell of any one of claims 1-15, which is derived from an embryonic stem cell (ESC), an induced pluripotent stem cell (iPSC), an epiblast stem cell (EpiSC), an epiblast cell (Epiblast), a blastocysts inner cell mass, a pluripotent stem cell induced from a somatic cell.

17. The pluripotent stem cell of any one of claims 1-16, wherein the pluripotent stem cell has theability to differentiate into of any one of endoderm, ectoderm and mesoderm cells.

18. The pluripotent stem cell of any one of claims 1-17, wherein the pluripotent stem cell is a mammalian pluripotent stem cell, e.g., a mouse, dog, pig, cow, sheep, monkey, or human pluripotent stem cell.

19. The pluripotent stem cell of any one of claims 1-18, wherein when the pluripotent stem cell is a female pluripotent stem cell, it comprises two X chromosomes, one of which is a normally activated X chromosome (Xa), and the other is a normally silent X chromosome (Xi).

20. The pluripotent stem cell of any one of claims 1-19, wherein the pluripotent stem cell is a cell line; e.g., the pluripotent stem cell is a formative pluripotent stem cell line.

21. An isolated cell population, comprising the pluripotent stem cell of any one of claims 1-20 or any combination thereof;
preferably, at least 50% (e.g., at least 60%, at least 70%, at least 80%, at least 85%, at least 90%, at least 95%, at least 98%, at least 99%, or about 100%) of the cells in the isolated cell population are the pluripotent stem cells of any one of claims 1-20.

22. A method for producing the pluripotent stem cell of any one of claims 1-20 or the isolated cell population of claim 21, comprising inhibiting epithelium-to-mesenchymal transition of the pluripotent stem cell, the pluripotent stem cell is derived from an embryonic stem cell (ESC), an induced pluripotent stem cell (iPSC) or an epiblast stem cell (EpiSC), a blastocyst inner cell mass, an epiblast cell, an early embryo from post-implantation to pre- and post-gastrulation.

23. The method for producing the pluripotent stem cell of any one of claims 1-20 or the isolated cell population of claim 21, comprising carrying out three-dimensional culture to the pluripotent stem cell, the pluripotent stem cell is derived from an embryonic stem cell (ESC), an induced pluripotent stem cell (iPSC) or epiblast stem cell (EpiSC), a blastocyst inner cell mass, an epiblast cell, an early embryo from post-implantation to pre- and post-gastrulation.

24. The method of claim 22 or 23, comprising carrying out three-dimensional culture of the pluripotent stem cell before and after gastrulation in medium, the medium is selected from basal medium supplemented with the following substances: a serum substitute, Activin A, bFGF, and a Wnt/β-catenin signal transduction inhibitor.

25. The method of claim 24, wherein the medium has one or more of the following characteristics:
(i) the serum substitute is selected from: KnockOut^{™} SR (abbreviated as KSR), N-2, B-27, Physiologix^{™} XF SR, StemSure^{™} serum substitute supplement, or any combination thereof;
(ii) the Wnt/β-catenin signal transduction inhibitor is selected from IWP-2, XAV939, Wnt-C59 (C59), IWP-L6, IWR-1-endo, ICG-001, KY1220, iCRT14;
(iii) the basal medium is selected from KnockOut DMEM, KnockOut DMEM/F12, DMEM, DMEM/F 12, neurobasal medium, and any combination thereof.

26. The method of claim 24 or 25, wherein the medium has one or more of the following characteristics:
(i) the content of the serum substitute is 0.1-20% (v/v), e.g., 0.1-10%;
(ii) the content of Activin A is 1-100ng/mL, e.g., 1-50ng/mL;
(iii) the content of bFGF is 1-100ng/mL, e.g., 1-50ng/mL;
(iv) the content of the Wnt/β-catenin signal transduction inhibitor is 1-50 µM, e.g., 1-25 µM.

27. The method of any one of claims 23-26, wherein the three-dimensional culture method comprises: culturing the pluripotent stem cell before and after gastrulation in presence of a three-dimensional scaffold to obtain three-dimensional culture.

28. The method of claim 27, wherein the three-dimensional scaffold is hydrogel;
preferably, the hydrogel comprises extracellular matrix;
preferably, the extracellular matrix comprises one or more of laminin, collagen, fibrin, hyaluronic acid or chitosan.

29. The method of claim 28, wherein the three-dimensional culturing comprises the steps of:
(1) mixing a single cell suspension of pluripotent stem cell before and after gastrulation with the hydrogel to obtain a gel-like mixture embedded with the pluripotent stem cell before and after gastrulation;
(2) cultivating the gel-like mixture in step (1) in the medium of any one of claims 24-26 to obtain three-dimensional culture.

30. The method of any one of claims 27-29, wherein the method further comprises isolation of cells from the three-dimensional culture, thereby obtaining the pluripotent stem cell;
preferably, the isolation comprises mechanical isolation and/or enzymatic digestion.

31. The method of claim 30, wherein the method further comprises passage of the obtained pluripotent stem cells;
preferably, the passage comprises inoculating the obtained pluripotent stem cells in the medium of any one of claims 24-26 for culturing.

32. A pharmaceutical composition, comprising the pluripotent stem cell of any one of claims 1-20 or the isolated cell population of claim 21, and a pharmaceutically acceptable carrier and/or excipient.

33. A method for in vitro production of an endoderm, ectoderm or mesoderm cell, or in vitro production of a tissue or an organ (e.g., a organoid), comprising: culturing the pluripotent stem cell of any one of claims 1-20, the isolated cell population of claim 21 or the pharmaceutical composition of claim 32 under a condition that allows differentiation of the pluripotent stem cell;
preferably, the cell is selected from the group consisting of a germ cell (e.g., a primordial germ cell), an epithelial cell, a connective tissue cell, a nerve cell (e.g., neuronal cell), an adipocyte, a pancreatic cell (e.g., insulin producing cell), a liver cell, a renal cell, a bone cell, a hematopoietic cell, an endothelial cell, a retinal cell, or a muscle cell (e.g., a cardiomyocyte).

34. Use of the pluripotent stem cell of any one of claims 1-20, the isolated cell population of claim 21 or the pharmaceutical composition of claim 32 for in vitro production of an endoderm, ectoderm or mesoderm cell or in vitro production of a tissue or an organ (e.g. an organoid);
preferably, the cell is selected from a germ cell (e.g., a primordial germ cell), an epithelial cell, a connective tissue cell, a nerve cell (e.g., neuronal cell), an adipocyte, a pancreatic cell (e.g., insulin producing cell), a liver cell, a renal cell, a bone cell, a hematopoietic cell, an endothelial cell, a retinal cell, and a muscle cell (e.g., a cardiomyocyte).

35. Use of the pluripotent stem cell of any one of claims 1-20, the isolated cell population of claim 21, or the pharmaceutical composition of claim 32, or the tissue or organ (e.g., the organoid) produced by the pluripotent stem cell, the isolated cell population or the pharmaceutical composition for being as a disease model and/or a drug screening model.

36. A method for regeneration or repair of a tissue or an organ, or for transplantation of a cell, a tissue or an organ, comprising: administering the pluripotent stem cell of any one of claims 1-20, the isolated cell population of claim 21, or the pharmaceutical composition of claim 32 to a subject in need thereof; or the method comprises: inducing in vitro differentiation and/or proliferation of the pluripotent stem cell of any one of claims 1-20 or the isolated cell population of claim 21, administering the differentiated and/or proliferated pluripotent stem cell or cell population, or the pharmaceutical composition comprising the differentiated and/or proliferated pluripotent stem cell or cell population to a subject in need thereof.

37. Use of the pluripotent stem cell of any one of claims 1-20, the isolated cell population of claim 21, or the pharmaceutical composition of claim 32, for (i) regeneration or repair of a tissue or an organ, or (ii) transplantation of a cell, a tissue or an organ, or in the preparation of a medicament for (i) regeneration or repair of a tissue or an organ, or (ii) transplantation of a cell, a tissue or an organ.
